Europäisches Patentamt

European Patent Office

Office européen des brevets

(19)

(11) Numéro de publication: **0 266 240**
A1

(12) **DEMANDE DE BREVET EUROPEEN**

(21) Numéro de dépôt: 87402157.9

(22) Date de dépôt: 28.09.87

(51) Int. Cl.⁴: **C 12 N 15/00**
C 12 P 17/18, C 12 N 1/00

(30) Priorité: 30.09.86 FR 8613603
18.05.87 FR 8706916

(43) Date de publication de la demande:
04.05.88 Bulletin 88/18

(84) Etats contractants désignés:
AT BE CH DE FR GB IT LI LU NL SE

(71) Demandeur: TRANSGENE S.A.
16, rue Henri Regnault
F-92400 Courbevoie (FR)

(72) Inventeur: Gloeckler, Rémi
22 rue de Stockholm
F-67000 Strasbourg (FR)

Speck, Denis
4, rue de Bitzen
F-67200 Eckbolsheim (FR)

Lemoine, Yves
4, rue des Allsiers
F-67100 Strasbourg-Neudorf (FR)

(74) Mandataire: Warcoin, Jacques et al
Cabinet Régimbeau 26, avenue Kléber
F-75116 Paris (FR)

(54) Clonage des gènes bioA, bioD, bioF, bioC, bioH de Bacillus sphaericus, vecteurs et cellules transformées et procédé de préparation de la biotine.

(57) La présente invention concerne notamment une séquence d'ADN correspondant à l'un des gènes suivants de la chaîne de biosynthèse de la biotine chez les bactéries : gène bioA, gène bioD, gène bioF, bioC et bioH.

Des vecteurs comportant ces séquences permettent de transformer d'autres microorganismes afin d'améliorer la production de biotine.

EP 0 266 240 A1

Bundesdruckerei Berlin

## Description

Clonage des gènes bioA, bioD, bioF,bioC, bioH de Bacillus sphaericus, vecteurs et cellules transformées et procédé de préparation de la biotine.

La présente invention concerne la préparation de la biotine par fermentation en utilisant la technique des ADN recombinants.

La biotine est une vitamine nécessaire pour l'homme, les animaux, les plantes et pour certains microorganismes.

Elle a été isolée du jaune d'oeuf et on en trouve dans la levure de bière, les céréales, différents organes, sous forme libre ou combinée à des protéines.

Cette vitamine a été notamment proposée comme régulateur du métabolisme cutané, notamment dans le traitement des dermites séborrhéiques chez l'homme.

Outre les différentes sources qui ont été rappelées précédemment, la biotine est synthétisée par certains microorganismes, en particulier des microorganismes du genre Bacillus tel que Bacillus sphaericus.

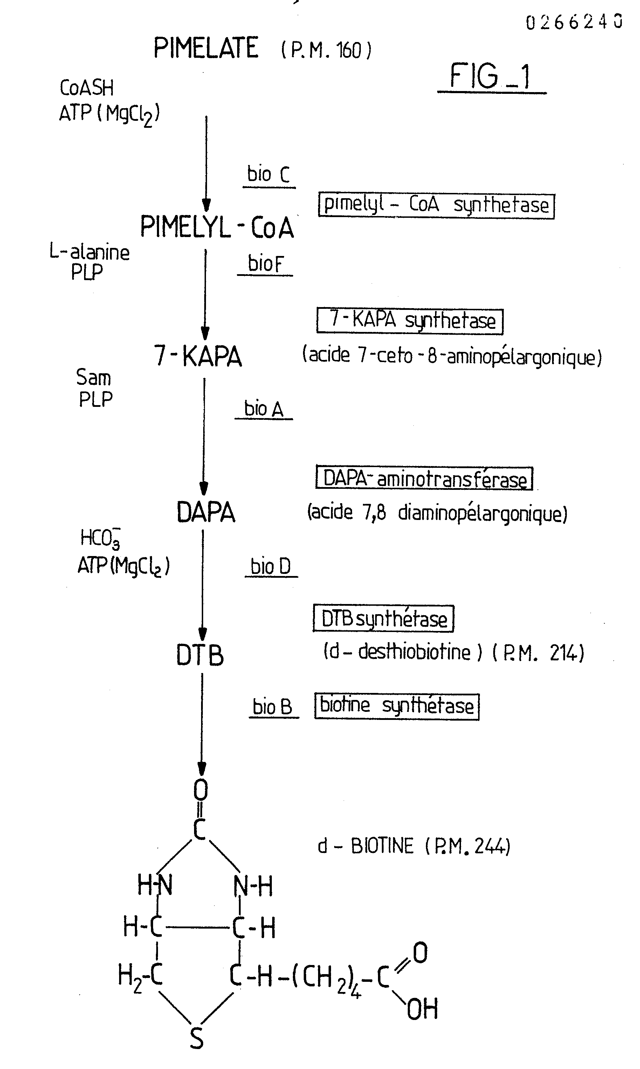

La figure 1 schématise la chaîne de biosynthèse de la biotine à partir de l'acide pimélique dans ce type de microorganisme. Cette chaîne de biosynthèse comprend 5 étapes enzymatiques différentes dans lesquelles les gènes mis en oeuvre sont dénommés successivement bioC, bioF, bioA, bioD et bioB.

L'étude systématique de la production de la biotine à partir de l'acide pimélique au cours de fermentations industrielles a montré que certains microorganismes, particulièrement ceux appartenant au genre Bacillus sphaericus, hyperproduisent des vitamères de la biotine de même que la biotine (on appellera ci-après "vitamères" les différents intermédiaires conduisant à la biotine).

Pour ces bactéries, parmi les précurseurs de la biotine, le DTB (desthiobiotine) représente le composant prédominant qui est produit en quantité beaucoup plus grande que la molécule finale : la biotine.

Il existe un fort contrôle de transcription réprimant la synthèse de la biotine qui dépend de la quantité de biotine présente dans le milieu de culture ; cette répression intervient dans E. coli de même que dans Bacillus sphaericus.

Mais aucune inhibition par "feed back" ne régule la biosynthèse de la biotine dans E. coli et jamais un tel contrôle n'a été décrit dans Bacillus sphaericus.

L'explication complète de la production d'une très grande quantité de DTB (pour une quantité faible de biotine) à partir de l'acide pimélique dans Bacillus sphaericus nécessite encore une étude de biologie moléculaire très importante sur l'organisation et la régulation de la voie de biosynthèse de la biotine dans cette bactérie.

Les études préliminaires de certaines des enzymes biosynthétiques extraites et semi-purifiées à partir de souches de B. sphaericus produisant le DTB ne rélèvent pas de différences marquantes avec les enzymes de la biotine bien connues dans E. coli.

Néanmoins, la production de biotine par fermentation industrielle de telles souches de Bacillus sphaericus (IFO 3525, NCIB 9370) pourrait devenir compétitive avec les procédés chimiques existants si le rendement en biotine était amélioré. De façon à atteindre ce but, il serait intéressant d'obtenir une hyperexpression constitutive de tous les gènes biosynthétiques de la biotine dans ces souches.

On a, bien entendu, décrit la sélection de souches de E. coli déréprimées, soit par leur résistance aux analogues de la biotine tels que l'alpha-déhydrobiotine, soit par sélection de mutants hyper sécréteurs de biotine. Il a également été décrit des mutations "cis dominantes" qui agissent de façon pléöïotrope sur la synthèse de tous les gènes de la biotine organisés en un un opéron bipolaire. Des mutations agissant en trans ont également été mentionnées, bien que, en plus de leur pouvoir d'abolir le contrôle de la transcription des gènes de la biotine, ells ont souvent des propriétés pléïotropes qui peuvent se répercuter sur la physiologie générale de la cellule.

Ces méthodes de sélection microbiologique traditionnelle peuvent aussi être appliquées aux souches de Bacillus sphaericus produisant du DTB, si l'on émet l'hypothèse que le contrôle moléculaire de la biosynthèse de la biotine et l'organisation générale des gènes de la biotine sont les mêmes que dans E. coli.

Au-delà de toutes ces hypothèses, l'utilisation de souches mutantes dans des fermentations industrielles importantes et en culture continue nécessite la sélection de mutants présentant un très faible taux de réversion, ce qui représente une tâche difficile étant donné l'absence actuelle de méthodes d'analyse génétique fine chez Bacillus sphaericus.

Une autre approche pourrait consister à cloner tous les gènes de la chaîne de biosynthèse de la biotine à partir d'une souche de Bacillus sphaericus produisant du DTB, puis à modifier, in vitro, la région de contrôle en 5' de façon à supprimer le contrôle de transcription.

En outre, le clonage et la manipulation in vitro de ces gènes permettraient l'étude de leur organisation générale et l'amélioration de leur transcription in vivo, par des techniques de génie génétique qui sont connues, impliquant en particulier l'utilisation de promoteurs forts, connus pour être fonctionnels dans les souches de Bacillus sphaericus.

La réintroduction de tous ces gènes hyperexprimés et qui ne seraient plus régulés par la biotine (mais qui seraient régulés de façon inductible en utilisant, par exemple, une induction par une augmentation de température ou par un substrat peu onéreux) dans la souche originale de Bacillus sphaericus pourrait alors être mise en oeuvre en utilisant les techniques connues de transformation, de transduction ou de

0 266 240

conjugaison-mobilisation.

On peut également envisager l'introduction de ces gènes dans des différents microorganismes qui sont connus pour être acceptables dans l'industrie alimentaire et qui sont perméables à l'acide pimélique, par exemple Bacillus subtilis, Saccharomyces cerevisiae ou des souches de Pseudomonas.

Enfin, la stabilisation de ces informations génétiques dans les microorganismes pourrait être réalisée par une intégration dirigée dans les chromosomes ou par une autosélection de plasmides tel que cela est décrit, par exemple, dans le brevet français no 84 12598.

Le clonage et la caractérisation du gène bioB de Bacillus sphaericus ont déjà été décrits dans les brevets JP-A-166 992/85 du 29 juillet 1985, JP-A-66 532/86 du 25 mars 1986 et JP-A-95 724/86 du 24 avril 1986.

Plus particulièrement, la présente invention concerne les séquences d'ADN codant pour l'enzyme produit de l'un des gènes suivants de la chaîne de biosynthèse de la biotine chez les bactéries :
- gène bioA
- gène bioD
- gène bioF
- gène bioC
- gène bioH.

Certaines de ces séquences d'ADN selon l'invention sont liées sous forme de "cluster" avec le gène bioB qui avait été précédemment identifié et l'ensemble couvre donc la plus grande partie de la chaîne de biosynthèse de la biotine.

Parmi les séquences d'ADN intéressantes, il faut citer les séquences d'ADN qui code pour les enzymes produits des gènes :
- bioB et bioD
- bioB, bioD et bioA
- bioB, bioD, bioA et bioF.

Ce type de séquence utilisé dans des vecteurs appropriés permet la préparation de la biotine à partir de ses différents vitamères.

Ceux-ci peuvent, comme cela sera décrit ci-après, être également préparés par fermentation en utilisant des vecteurs qui expriment les séquences d'ADN qui codent pour la ou les enzymes produits des gènes :
- bioF et bioC
- bioF et bioH
- bioF, bioC et bioH,
ainsi que les séquences qui codent, en plus des séquences précédentes, pour les gènes :
- bioA
- bioA et bioD, ou
- bioA, bioB et bioD.

Bien que les séquences d'ADN mentionnées précédemment puissent être d'origine diverse, on préférera utiliser les séquences provenant d'une souche de Bacillus, en particulier d'une souche de Bacillus sphaericus.

Comme cela a été indiqué précédemment, il est particulièrement intéressant que ces séquences d'ADN soient dépourvues des séquences naturelles assurant le contrôle de la transcription des enzymes de la voie de biosynthèse de la biotine chez la bactérie d'origine afin d'abolir la régulation naturelle due à la biotine et de placer ces séquences d'ADN sous le contrôle d'éléments choisis, qui assureront leur transcription efficace dans la souche hôte.

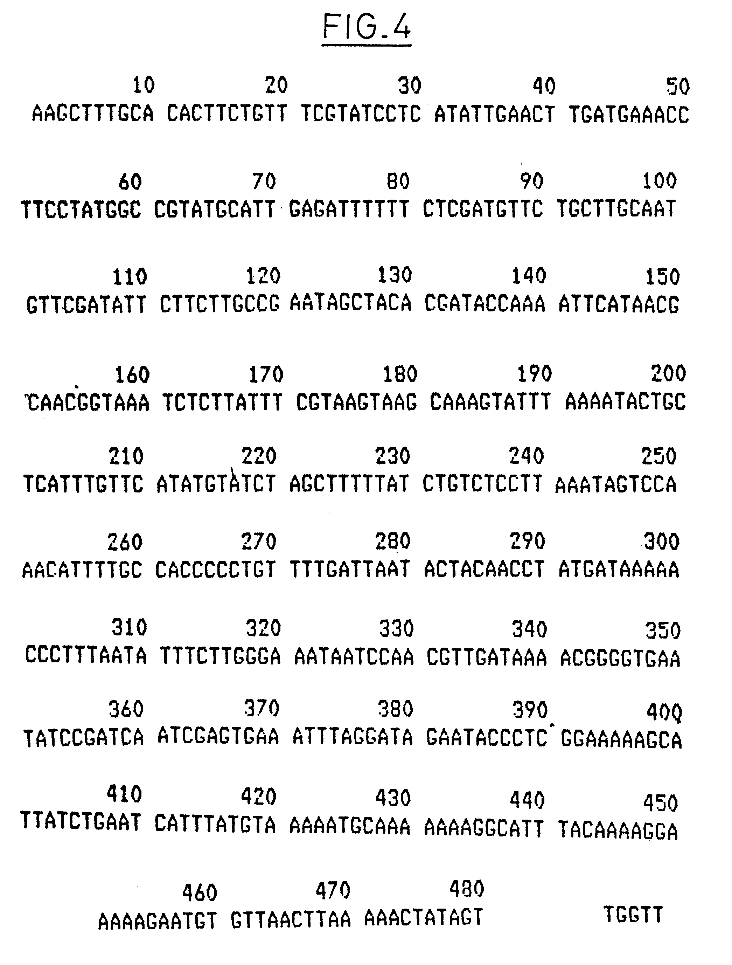

L'invention concerne, en particulier, tout ou partie des séquences qui sont représentées dans les figures 4 à 8 et 17 à 19 et qui codent pour l'un des gènes mentionnés précédemment.

Les séquences d'ADN selon la présente invention peuvent être utilisées de différentes façons.

De préférence, les séquences d'ADN en cause seront portées par un vecteur plasmidique susceptible d'assurer la transformation d'une bactérie et comportant l'ensemble des éléments assurant l'expression des gènes correspondants.

Ce plasmide pourra, comme cela a été indiqué, être de type autonome et auto-réplicable ou bien, au contraire, prévu pour assurer son intégration dans le chromosome de la souche hôte.

Pour ce faire, les différentes technologies à mettre en oeuvre sont connues ou seront décrites dans les exemples.

Ainsi, dans le cas d'un vecteur d'intégration, celui-ci devra comporter au moins une séquence homologue d'une séquence présente dans le génome de la souche à transformer, ce qui permettra d'assurer l'intégration chromosomique. Il pourra évidemment s'agir, soit d'une séquence homologue correspondant à une séquence génomique naturelle, soit d'une séquence homologue introduite par un autre vecteur plasmidique d'intégration.

Lorsque le vecteur est de type autonome et auto-réplicable, il comportera une origine de réplication efficace dans la cellule hôte.

De même, ces différents vecteurs plasmidiques pourront comporter des éléments assurant une sélection tels qu'un gène de résistance à un antibiotique et/ou un gène marqueur, sous la dépendance d'un promoteur de la souche à transformer.

Parmi les cellules pouvant servir de souche hôte, il faut mentionner plus particulièrement les bactéries, notamment des genres Escherichia, Bacillus et Pseudomonas ainsi que les levures, en particulier les levures du genre Saccharomyces.

3

Parmi les cellules hôtes particulièrement intéressantes, il faut citer Bacillus sphaericus, Bacillus subtilis et Escherichia coli.

Enfin, les souches plus particulièrement intéressantes pour être transformées par les séquences d'ADN selon la présente invention sont les souches qui ont déjà été transformées par des vecteurs assurant l'expression des autres gènes de cette voie de biosynthèse, c'est-à-dire les gènes F, A, D et B tels qu'ils sont décrits dans la présente demande.

Dans ces conditions, l'introduction des gènes bioC et bioH dans la bactérie permet à cette bactérie de synthétiser la biotine à partir du premier vitamère de la chaîne, à savoir le pimélate, ce qui présente un intérêt économique important sur le plan industriel.

Dans le cadre de la présente invention, le vecteur plasmidique d'intégration est plus particulièrement le plasmide pTG475 qui sera décrit ci-après et qui comporte notamment un promoteur inductible.

Lorsque le vecteur comporte une origine de réplication autonome, les séquences d'ADN codant pour les enzymes mentionnées précédemment seront, de préférence, flanquées d'éléments de contrôle assurant leur expression dans la souche hôte ; il s'agira en particulier, à l'extrémité 5′, d'un promoteur fort, efficace dans ladite souche et éventuellement d'autres éléments tels qu'une séquence de terminaison lorsque la souche hôte sera une levure ou tout autre microorganisme où une telle séquence est nécessaire.

La présente invention concerne également les cellules transformées par les plasmides vecteurs selon l'invention. Parmi ces cellules, il faut citer plus particulièrement les bactéries, notamment des genres Bacillus, Escherichia ou Pseudomonas, mais également les levures, en particulier du genre Saccharomyces.

Parmi les souches qui peuvent être transformées par ces vecteurs, il faut citer les souches qui produisent déjà de la biotine ou l'un de ses vitamères.

Enfin, la présente invention concerne un procédé de préparation de la biotine dans lequel on fait fermenter un milieu de croissance contenant au moins de l'acide pimélique, ou l'un des vitamères de la biotine, par des cellules telles qu'elles ont été décrites précédemment, qui soient perméables soit à l'acide pimélique, soit auxdits vitamères de la biotine et en ce qu'on récupère la biotine produite.

Le procédé selon l'invention peut être mis en oeuvre avec différentes variantes.

En particulier, il est possible de préparer le vitamère in situ à l'aide de cellules transformées par un vecteur selon l'invention, notamment une souche transformée contenant les gènes bioF, bioH et bioC peut être capable de produire KAPA à partir de l'acide pimélique, la transformation de ce KAPA en biotine étant effectuée par une souche portant les gènes complémentaires D, A et B par exemple.

Il est possible de prévoir deux fermentations successives ou bien une co-fermentation si les souches en présence s'y prêtent.

Il est également possible de prévoir la complémentation d'une souche qui ne possède qu'une partie des gènes en cause, ou encore de transformer une souche qui est déjà productrice de biotine pour la rendre surproductrice.

D'autres caractéristiques et avantages de la présente invention apparaîtront à la lecture des exemples ci-après décrits en se référant aux figures sur lesquelles :

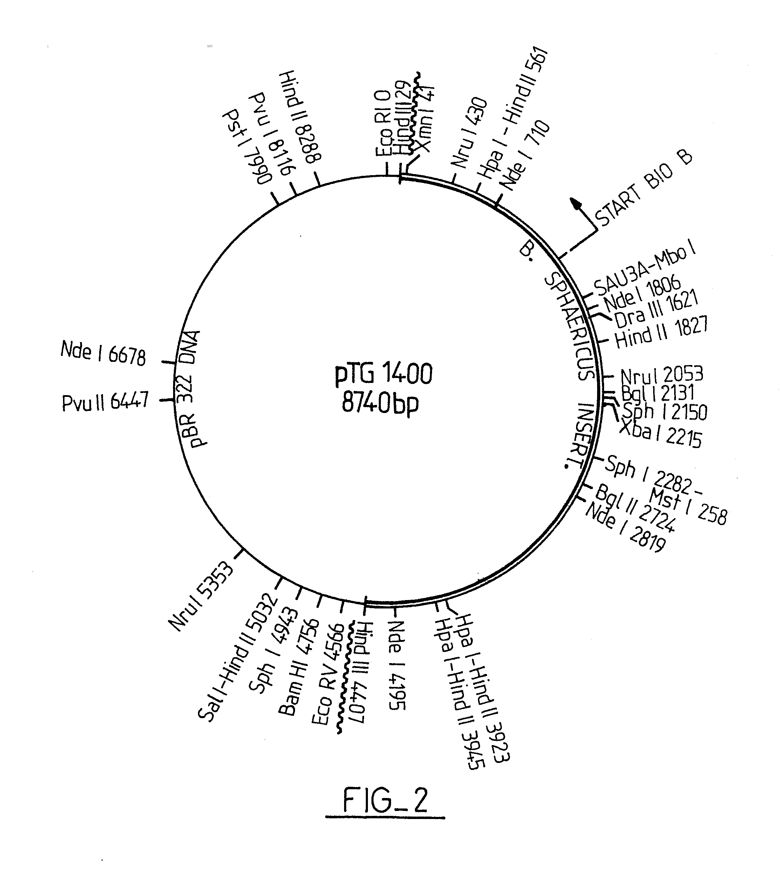

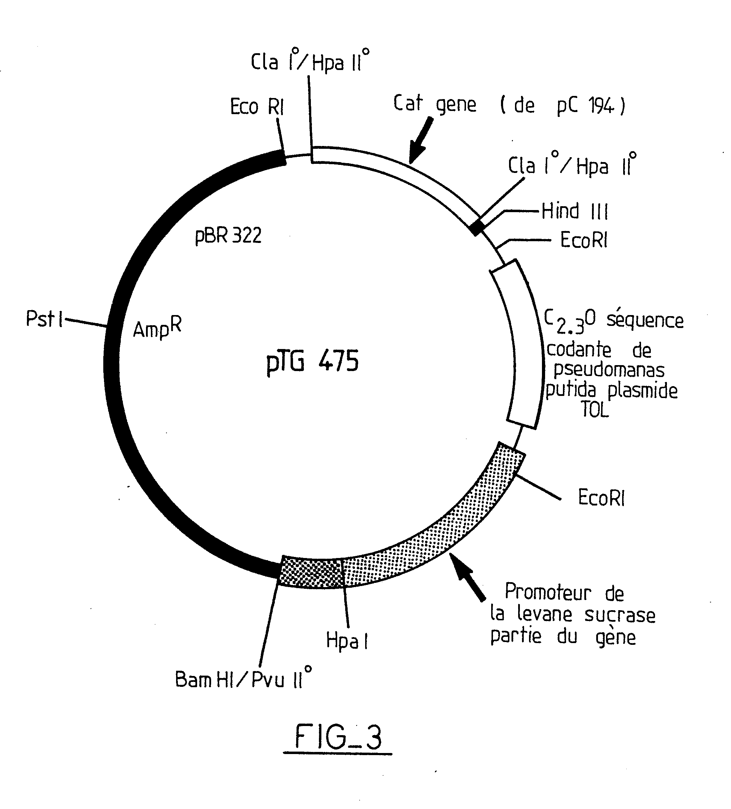

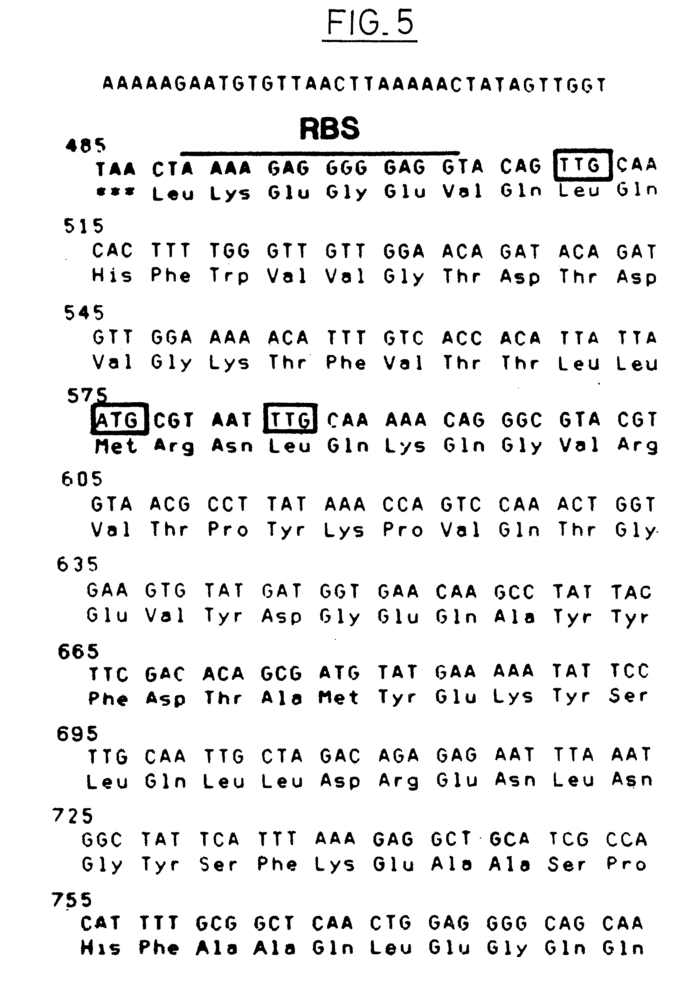

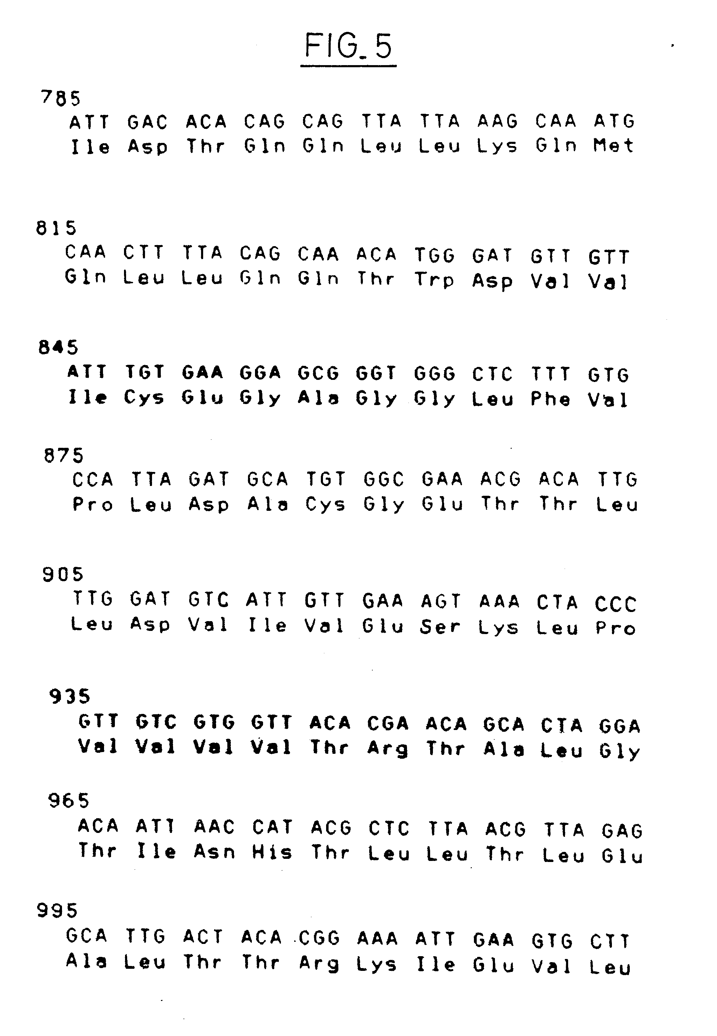

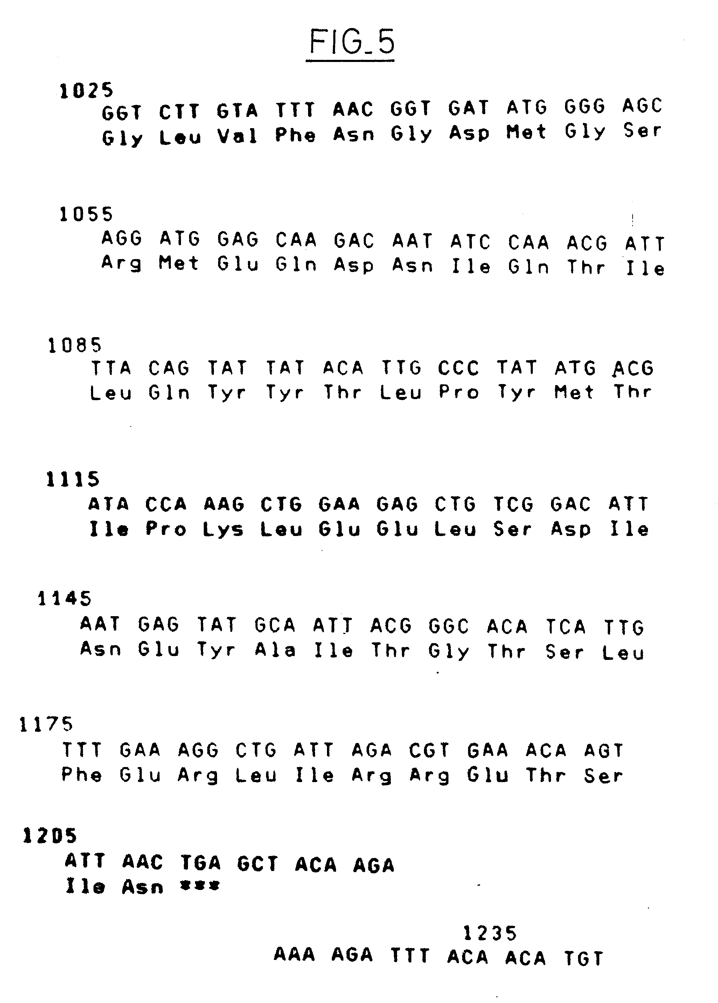

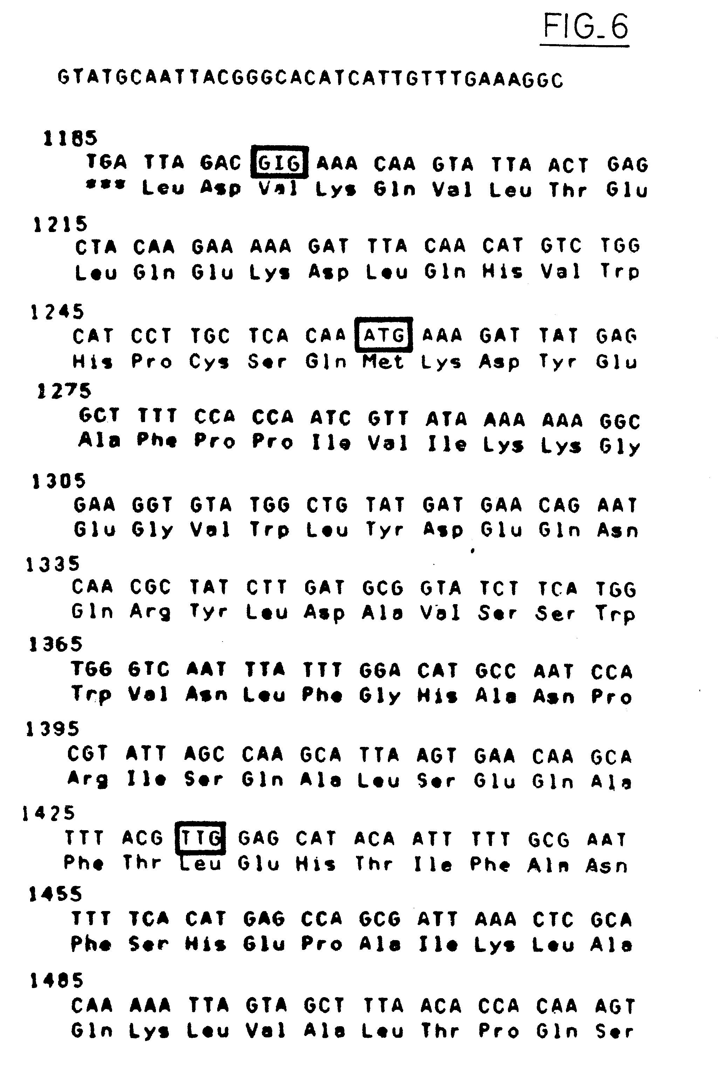

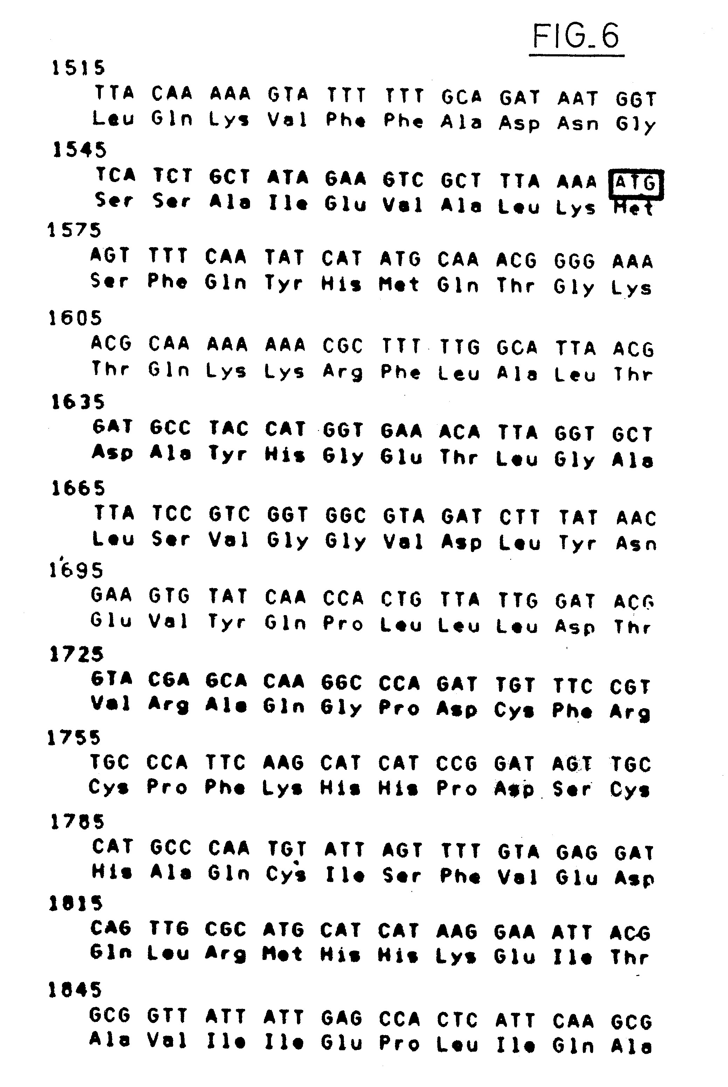

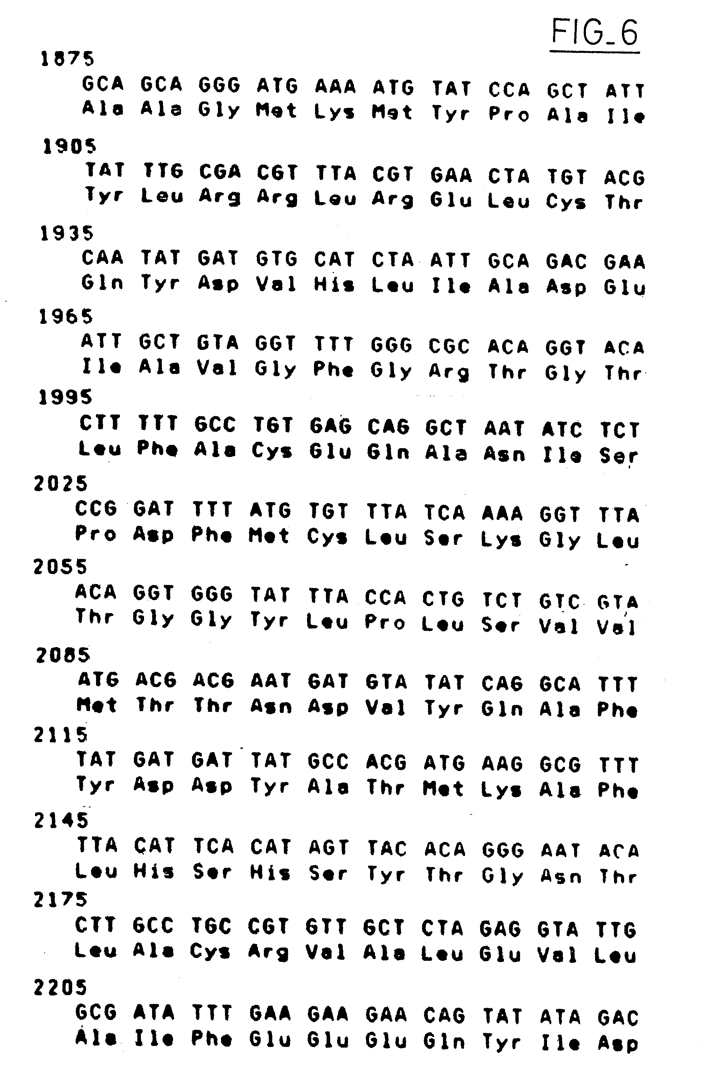

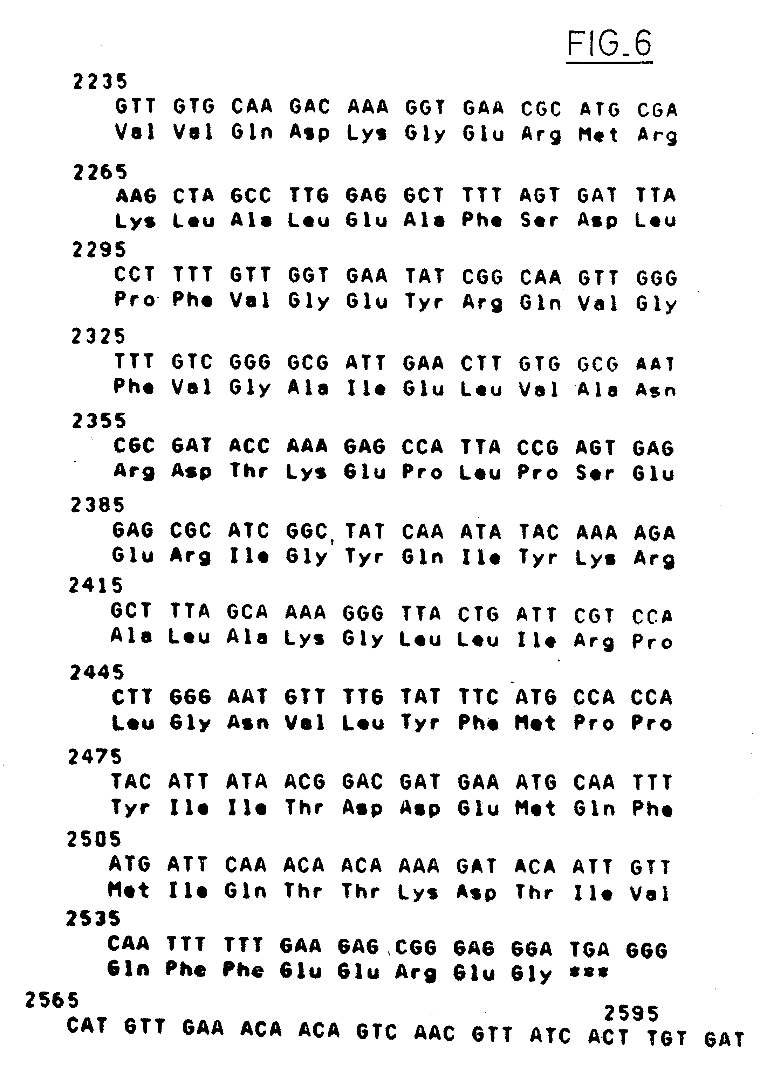

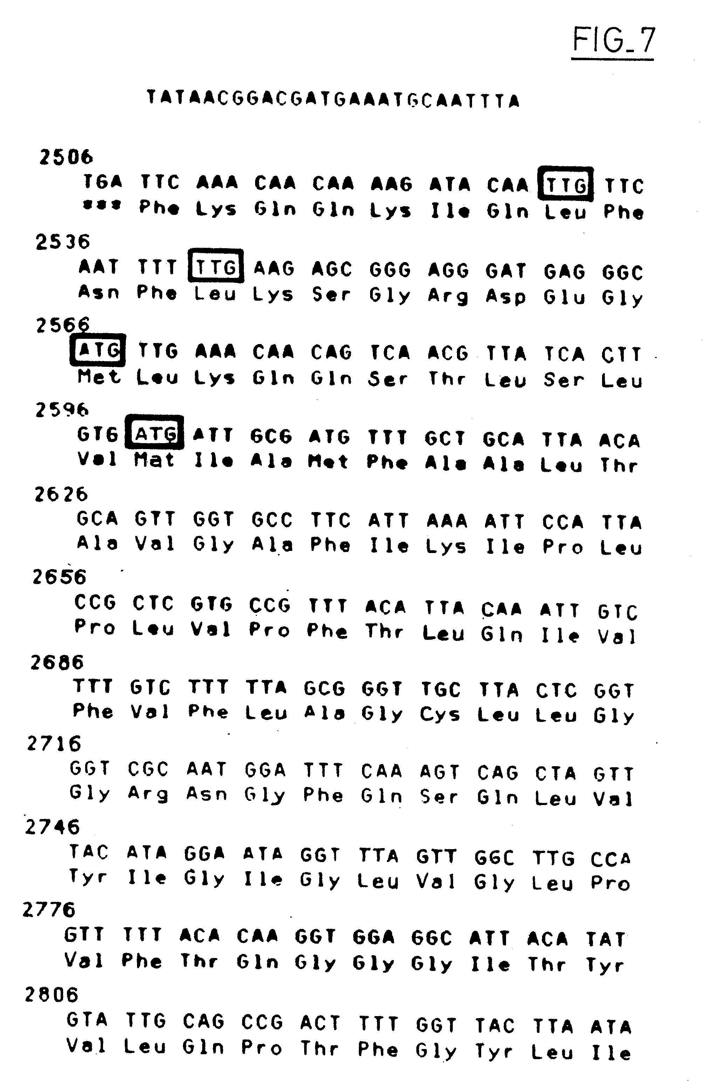

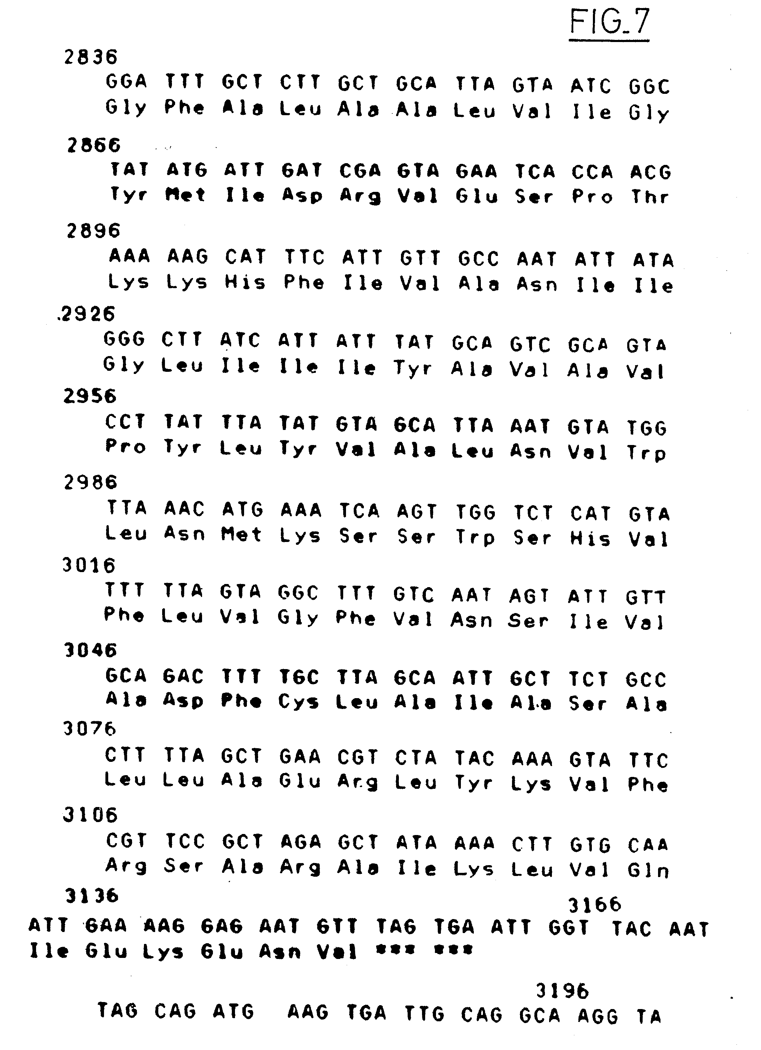

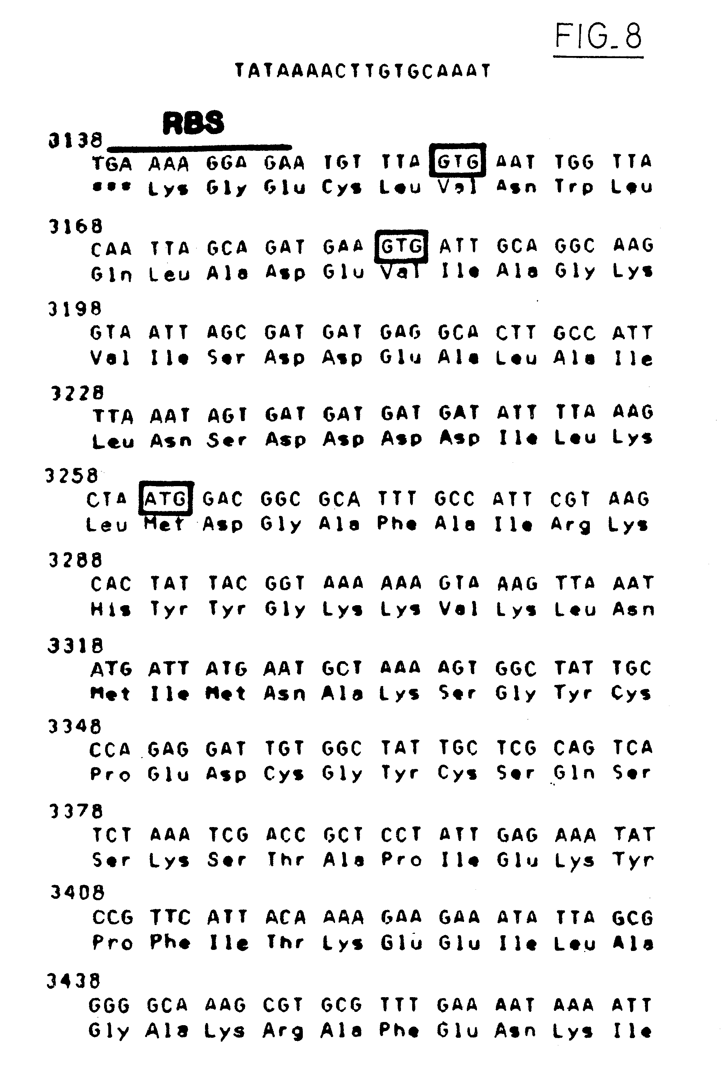

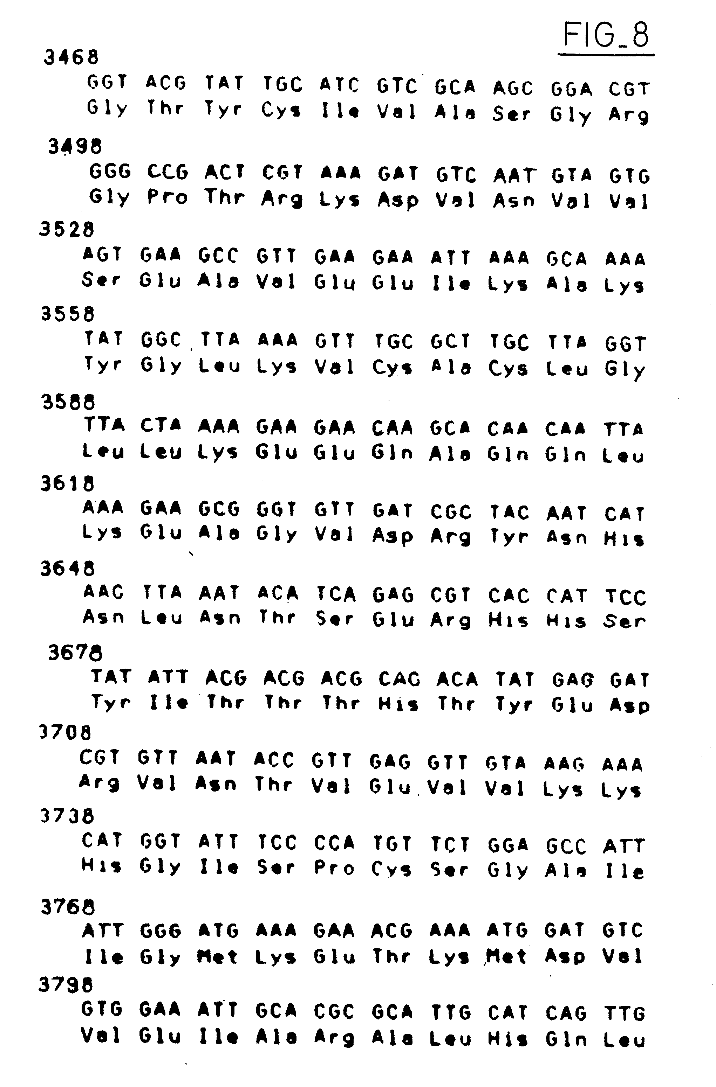

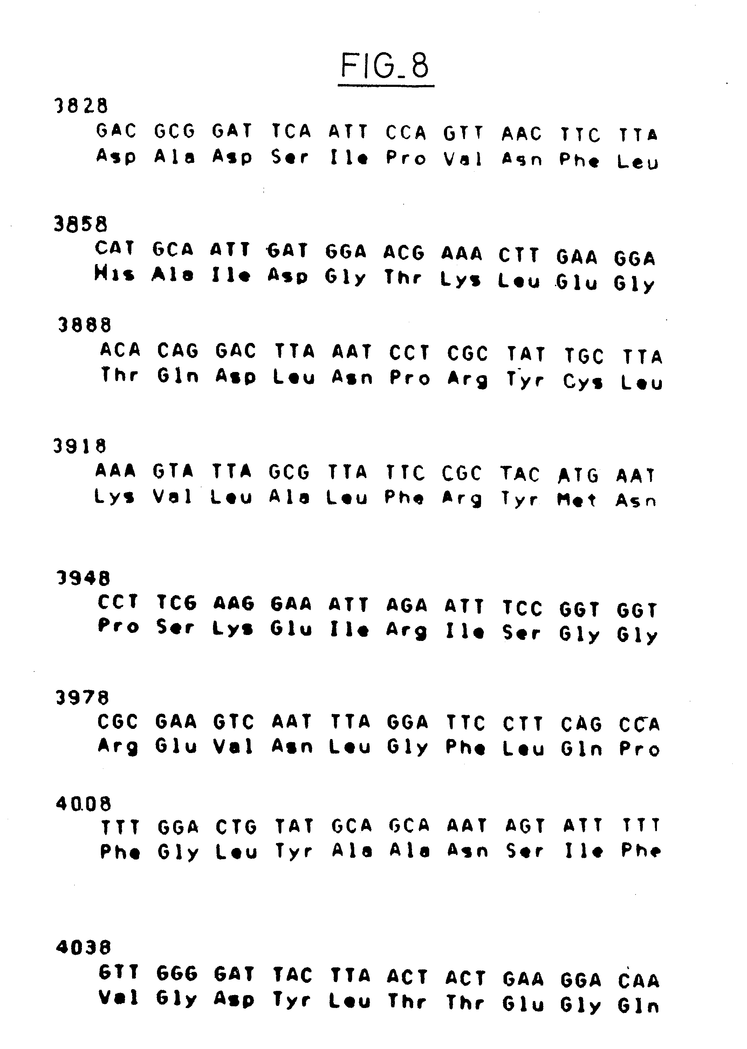

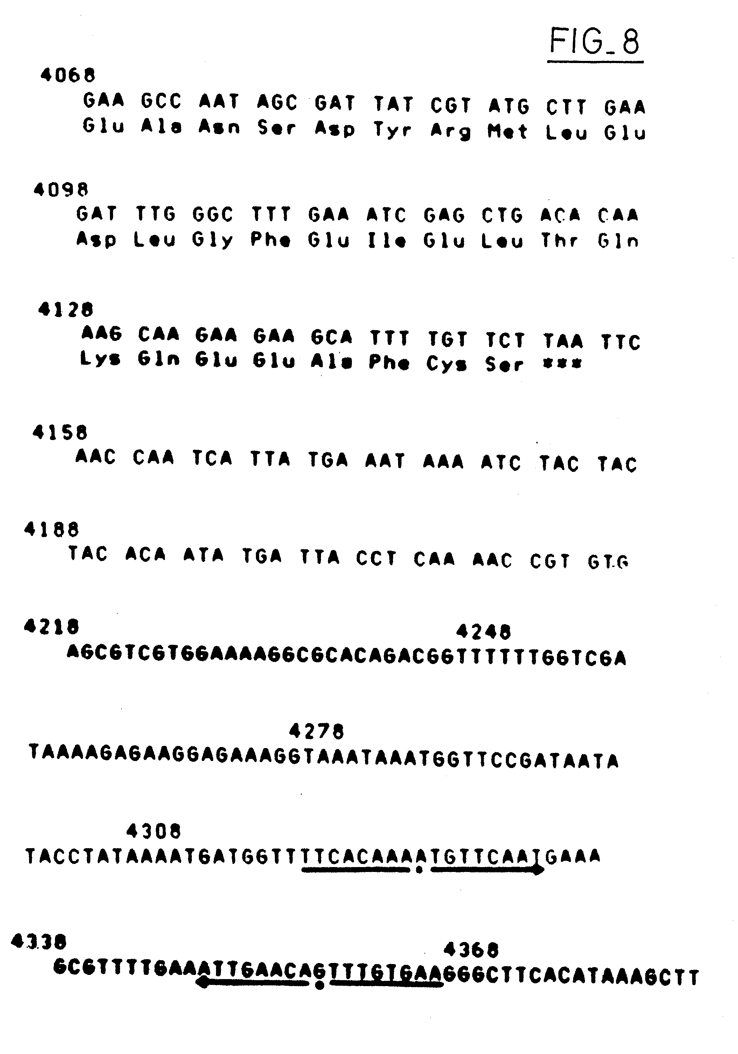

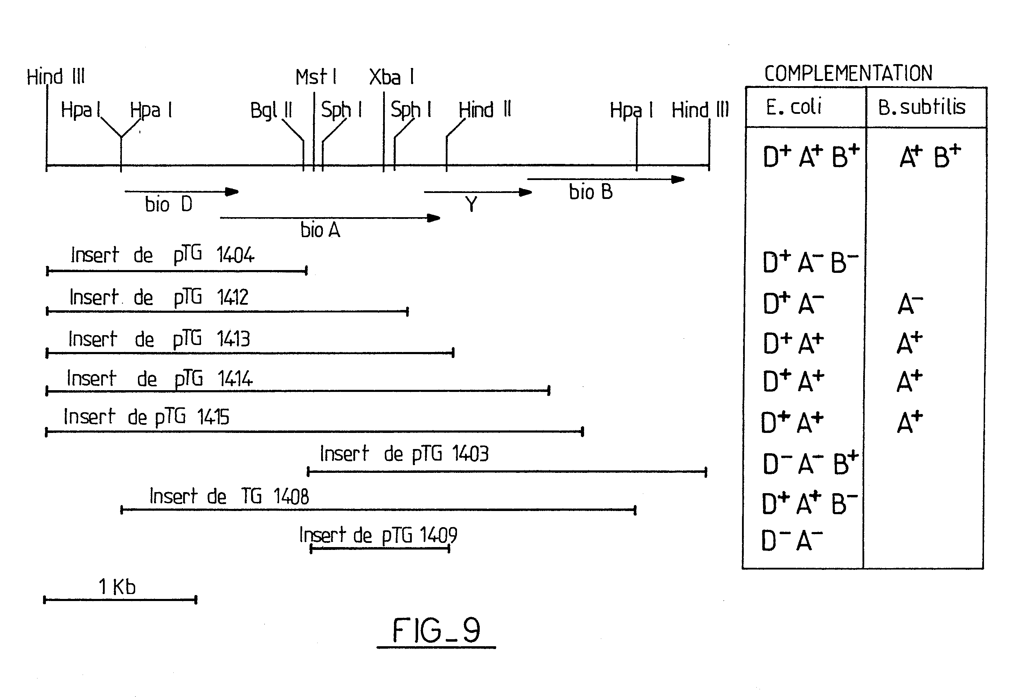

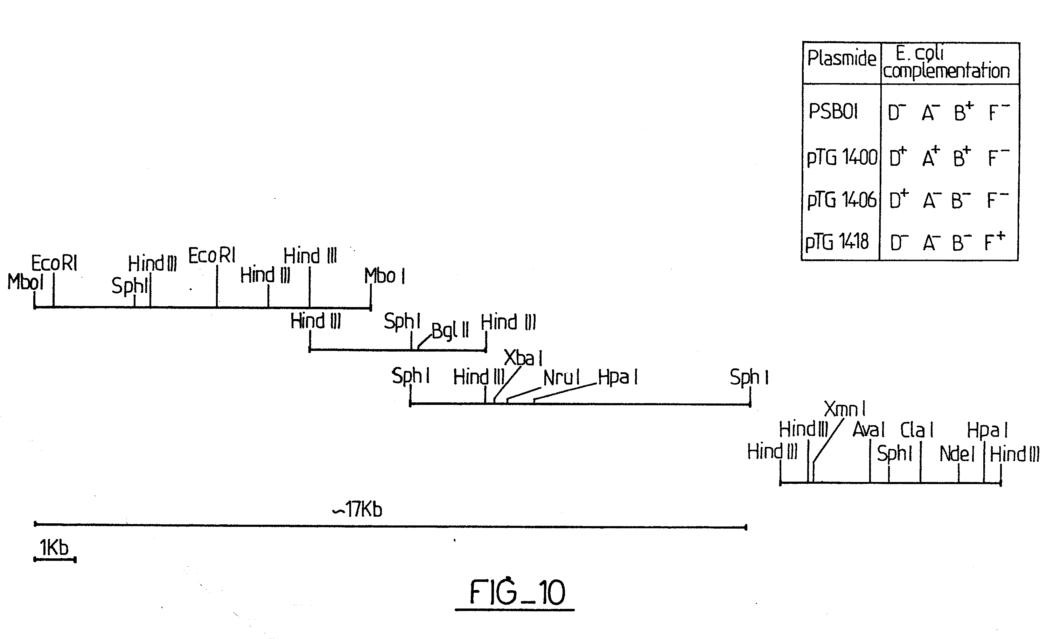

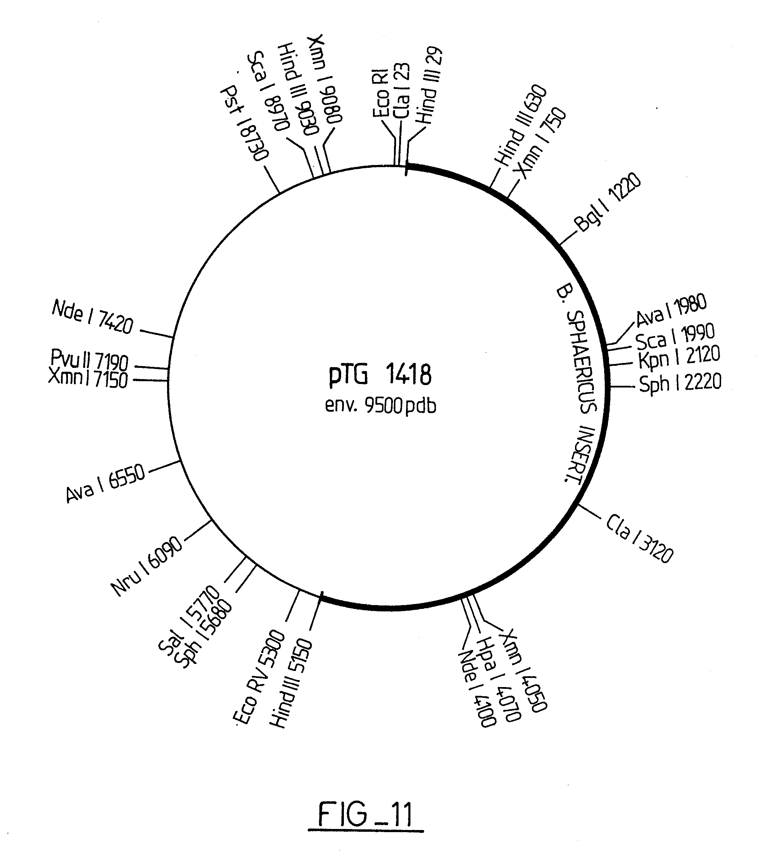

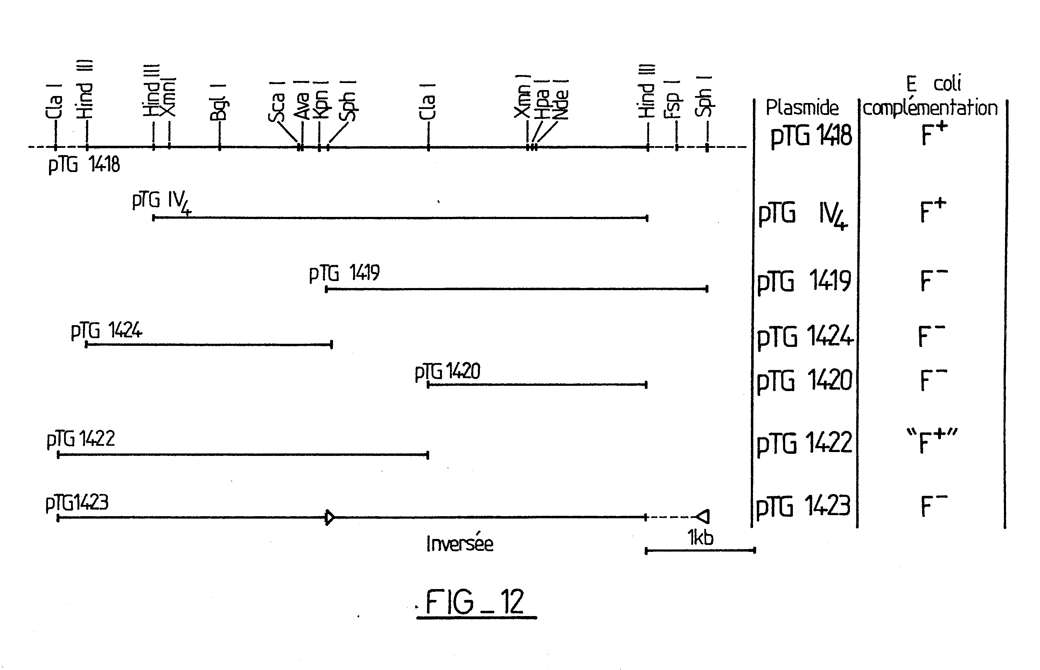

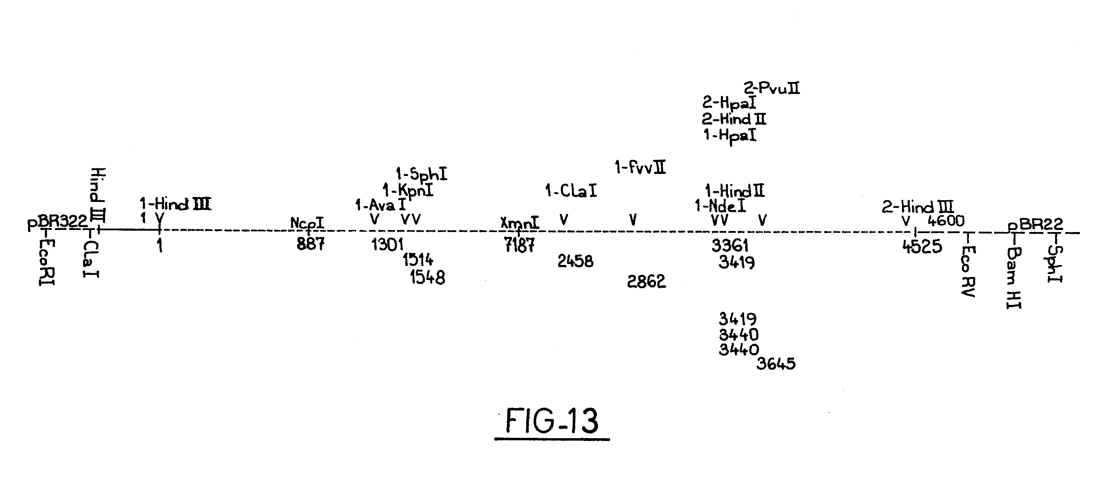

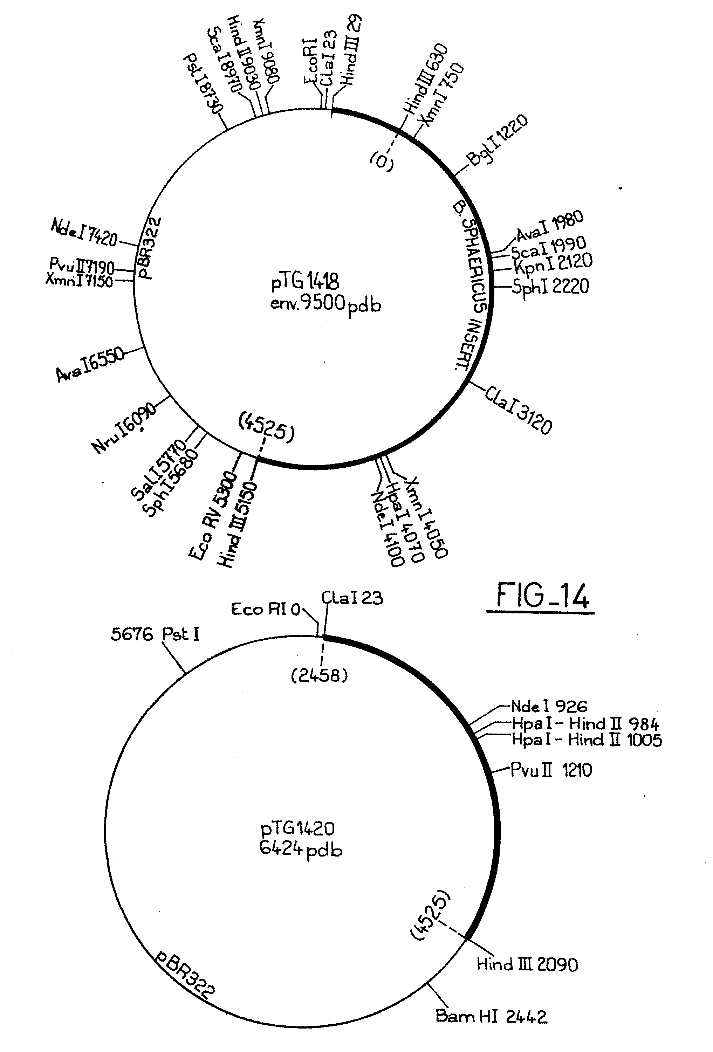

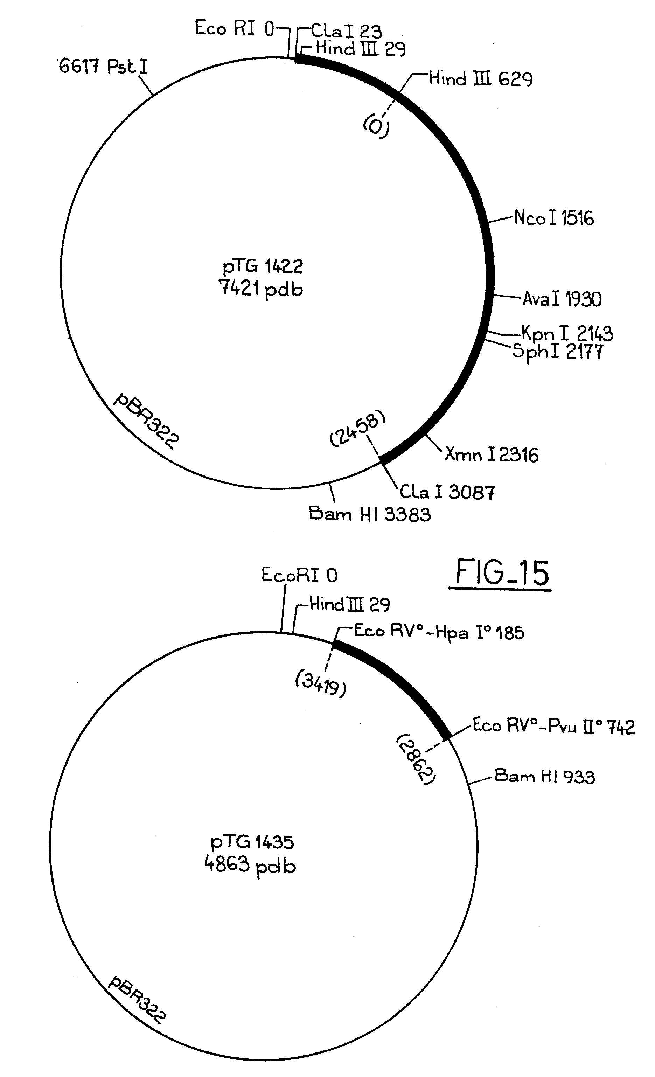

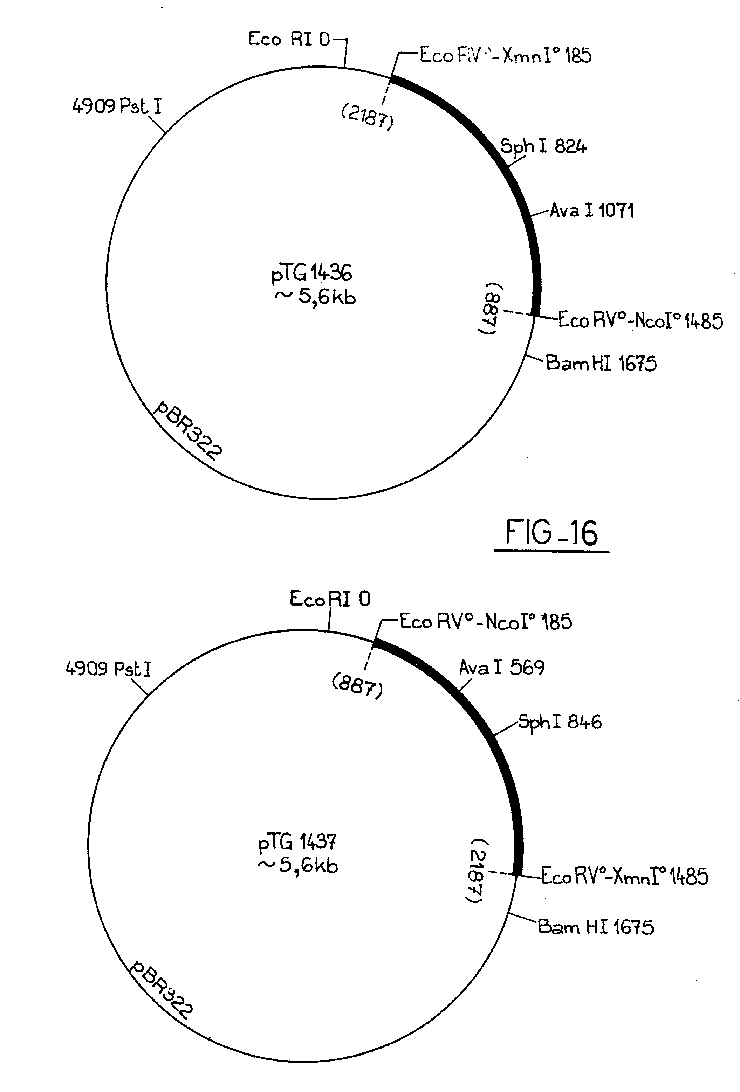

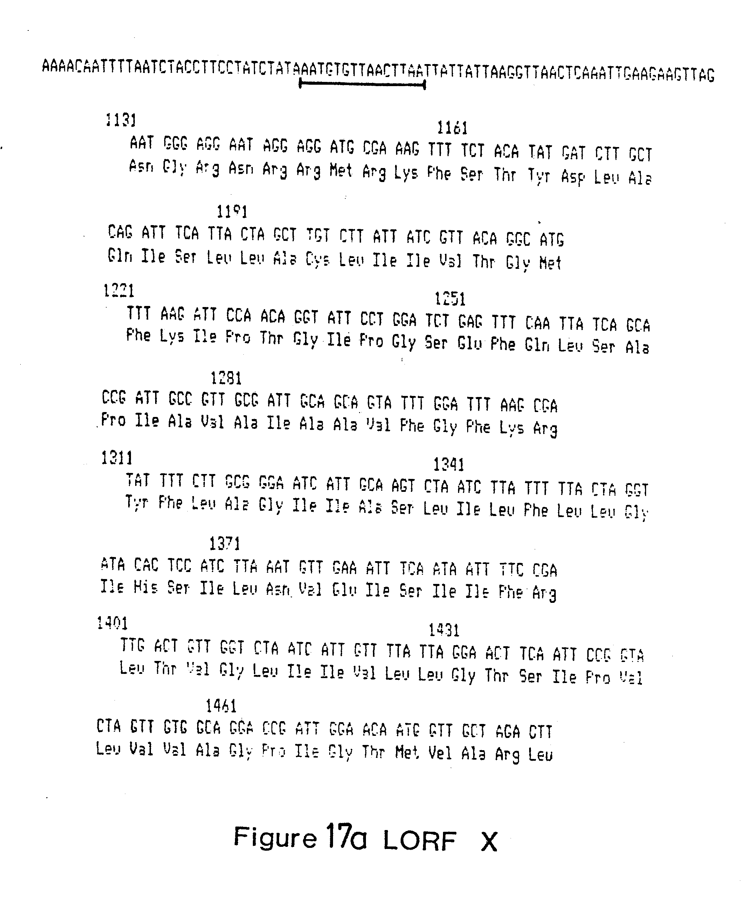

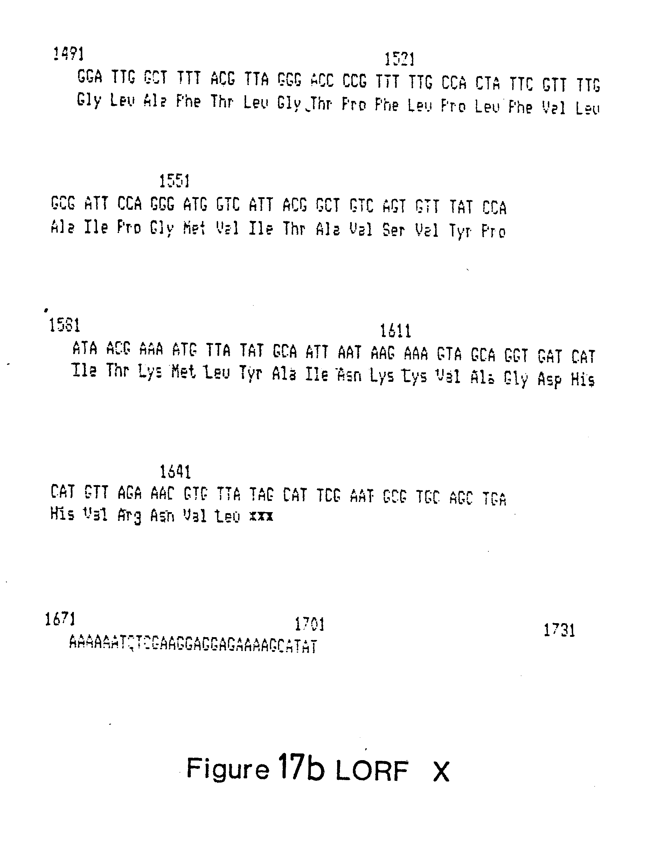

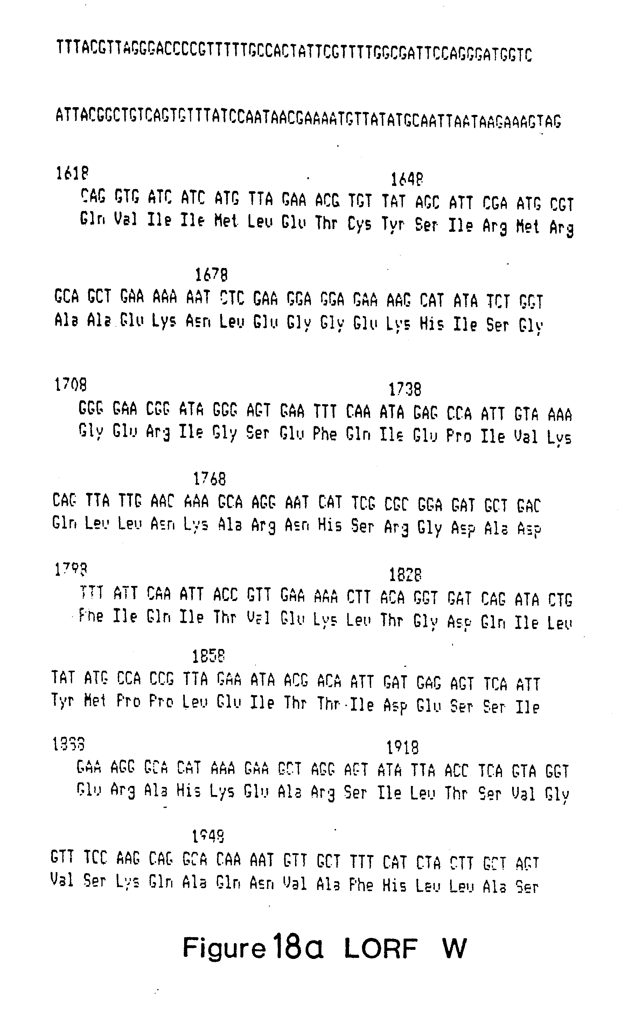

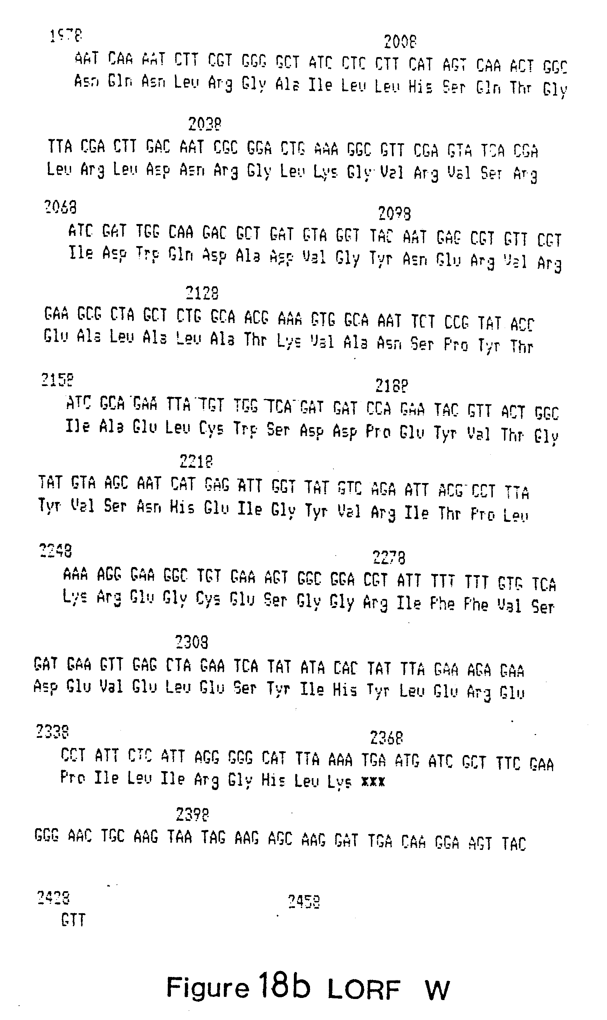

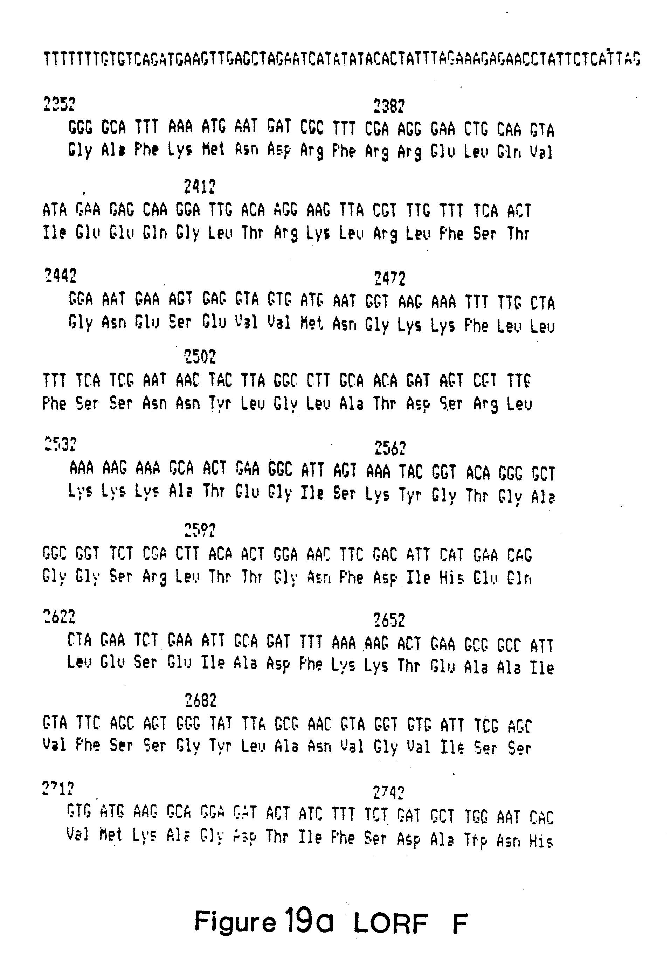

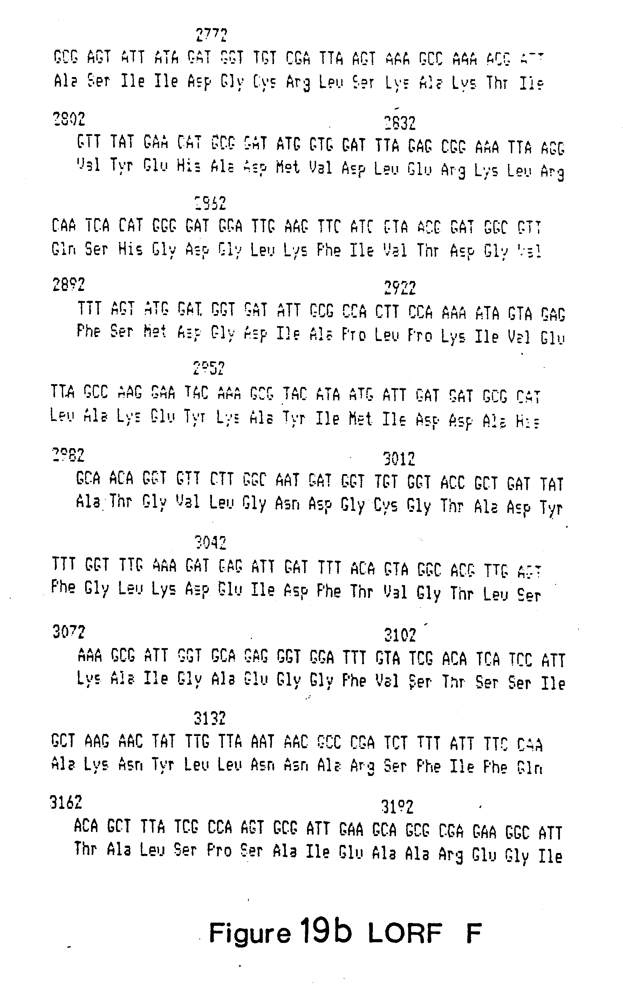

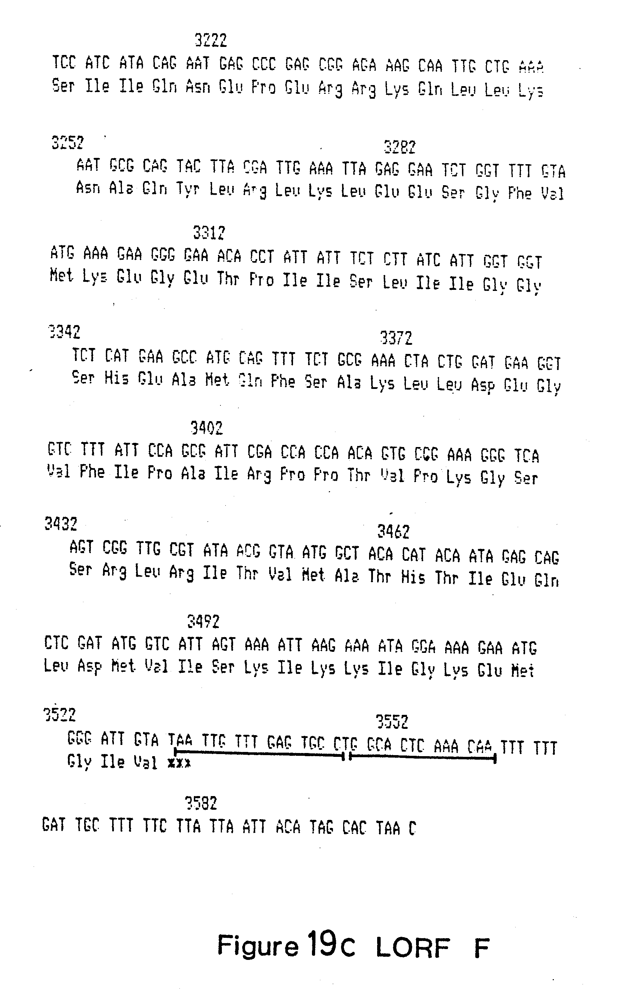

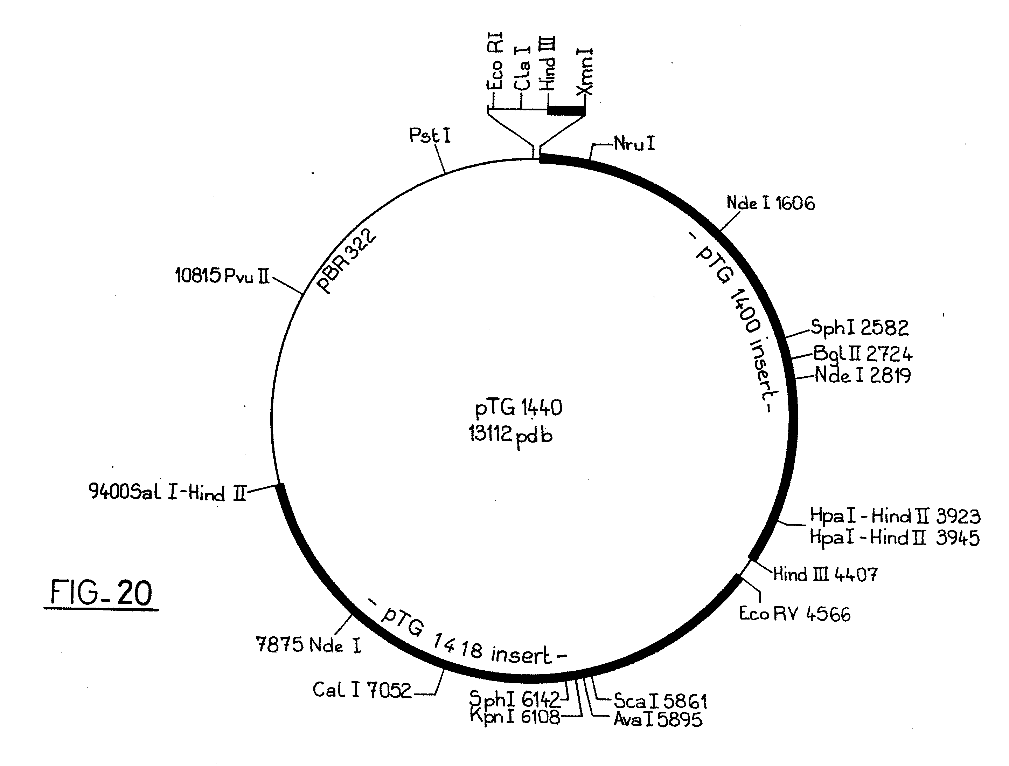

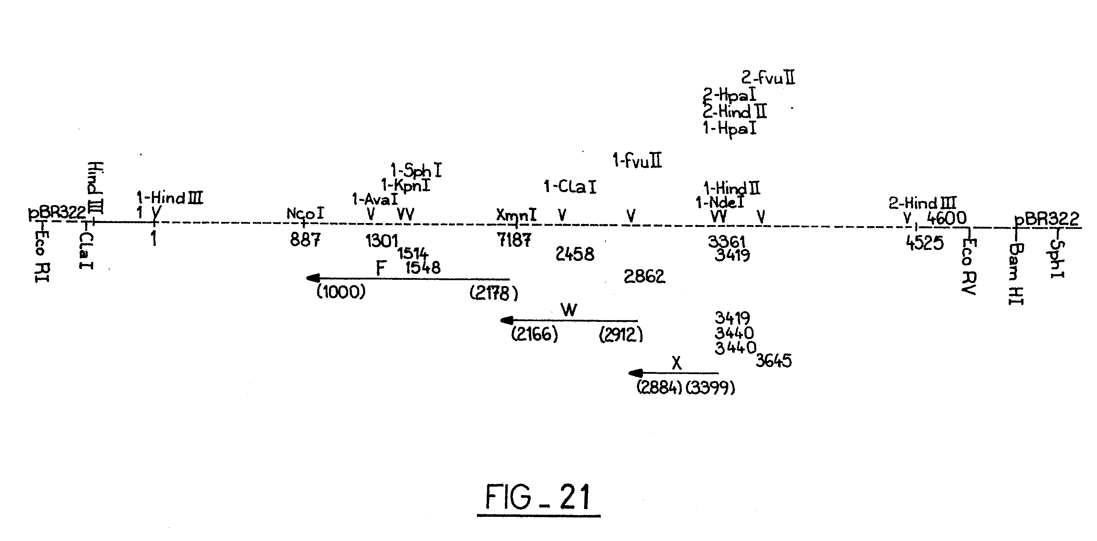

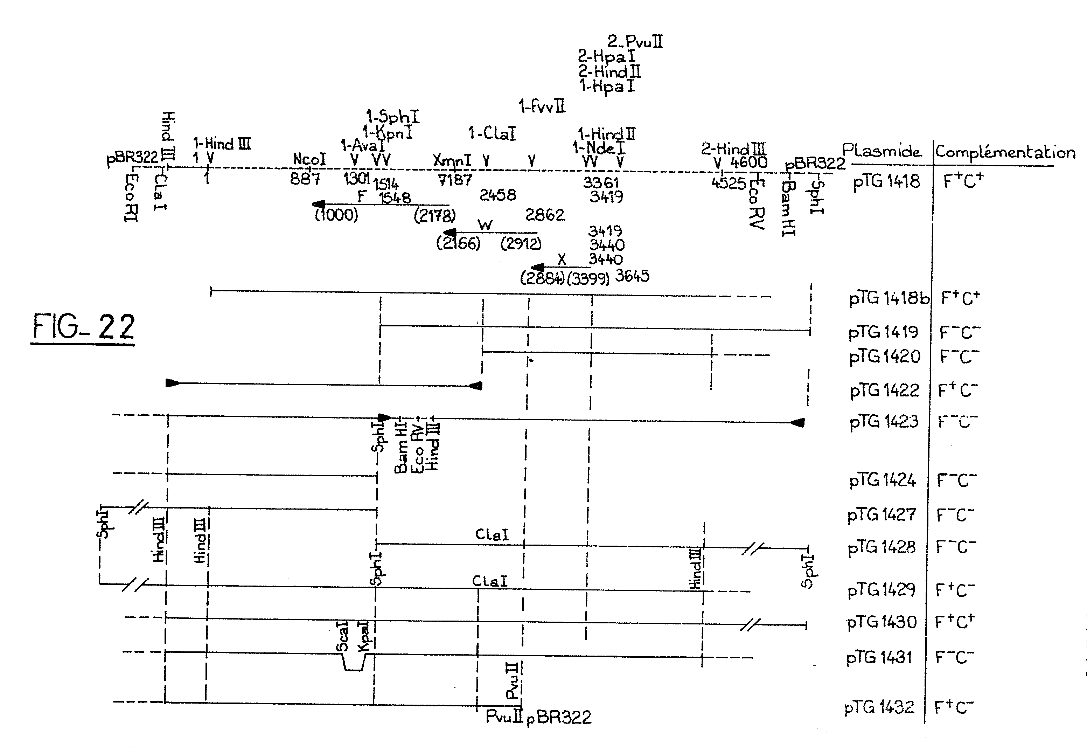

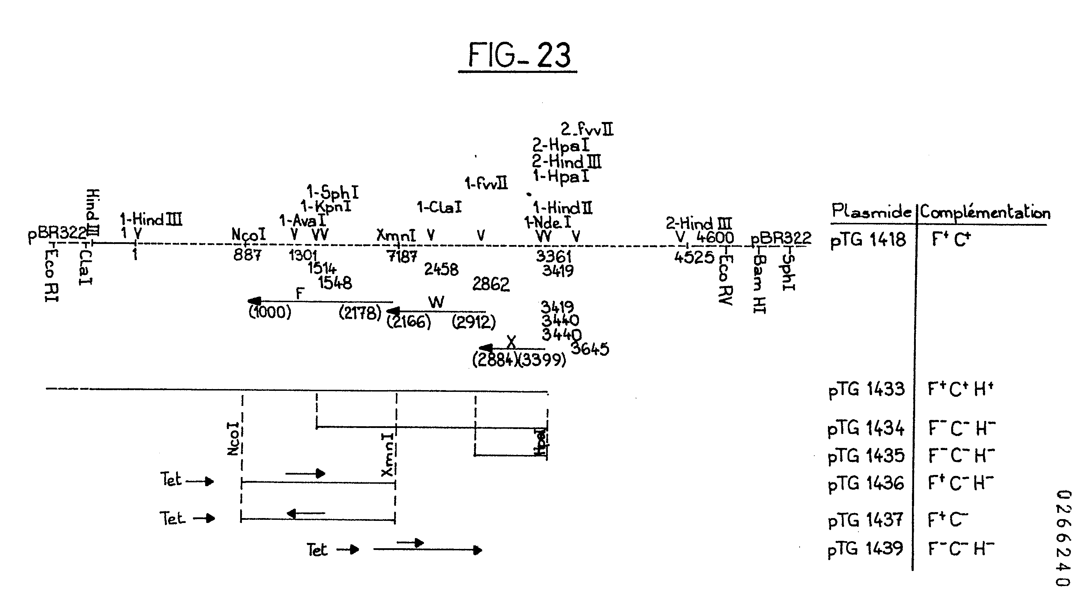

. la figure 1 représente la chaîne de biosynthèse de la biotine,
. la figure 2 schématise le plasmide pTG1400,
. la figure 3 schématise le plasmide pTG475,
. la figure 4 représente la séquence non codante en amont de la séquence LORF no 1,
. la figure 5 représente la séquence LORF no 1 correspondant au gène bioD,
. la figure 6 représente la séquence LORF no 2 correspondant au gène bioA,
. la figure 7 représente la séquence LORF no 3 correspondant au gène Y,
. la figure 8 représente la séquence LORF no 4 correspondant au gène bioB,
. la figure 9 schématise l'étude de complémentation de pTG1400,
. la figure 10 schématise l'étude de complémentation entre différents plasmides,
. la figure 11 schématise la structure du plasmide pTG1418,
. la figure 12 schématise l'essai de complémentation de pTG1418,
. la figure 13 représente la carte de restriction de l'insert de B. sphaericus utilisé dans les plasmides suivants :
. la figure 14 schématise les plasmides pTG1418 et pTG1420,
. la figure 15 schématise les plasmides pTG1422 et pTG1435,
. la figure 16 schématise les plasmides pTG1436 et pTG1437,
. la figure 17 représente la séquence LORF X,
. la figure 18 représente la séquence LORF W,
. la figure 19 représente la séquence LORF F,
. la figure 20 schématise le plasmide pTG1440,
. la figure 21 représente la carte de restriction de l'insert du plasmide pTG1418,
. les figures 22 et 23 représentent les différents plasmides dérivés du pTG1418 utilisés dans les tests de complémentation.

Pour des raisons de simplification, les séquences d'ADN et les structures des plasmides ont été représentées dans les dessins annexés, il est toutefois entendu qu'elles doivent être considérées comme faisant partie intégrante de la présente description.

EXEMPLE 1

Clonage par complémentation des gènes bioA et bioD de Bacillus sphaericus IFO 3525 et mise en évidence de leur liaison avec bioB

a) Dans E. coli

Bacillus sphaericus IFO 3525 est mis en culture dans 200 ml de milieu de culture PAB (DIFCO Bacto antibiotique, milieu 3, 17,5 g/l) à 37°C pendant 17 heures. Les bactéries sont récupérées par centrifugation et l'ADN total est alors extrait à partir des cellules par la méthode de Saito (Saito et al. BBA 1963, $\underline{72}$, 619-629). Une quantité de 450 µg d'ADN pur est obtenue.

20 µg de l'ADN total sont mis en restriction totale avec HindIII (3 U/µg d'ADN). pBR322 est traité avec la phosphatase alcaline après avoir été complètement digéré par HindIII.

Les plasmides recombinants hybrides sont obtenus en mélangeant l'ADN génomique digéré par HindIII (2 µg) et pBR322 traité comme précédemment (1 µg) avec 2 unités de ligase $T_4$ (Boehringer Mannheim) dans 50 µl du tampon réactionnel contenant NaCl 30 mM, Tris HCl pH 7,5 30 mM, $MgCl_2$ 10 mM, EDTA pH 8 0,2 mM, DTT 2 mM, ATP pH 7 0,5 mM et BSA 0,1 mg/ml. L'incubation est effectuée à 14°C pendant 16 heures. Des aliquotes du mélange de ligation sont alors ajoutées dans une expérience de transformation (Cohen et al. (1972) PNAS $\underline{69}$, 2110-2114) en utilisant la souche E. coli C600 $r_K- m_K+$ et en sélectionnant les souches pour leur résistance à l'ampicilline (100 µg/ml) sur milieu LB.

4 "pools" différents d'ADN plasmidique sont alors extraits, chacun correspondant à une moyenne de $10^4$ clones individuels sur les boîtes de transformation.

Différents mutants bio de E. coli sont alors transformés avec ces "pools" d'ADN et les transformés sont sélectionnés soit en présence d'ampicilline (100 µg/ml) sur milieu LB soit pour la résistance à cet antibiotique et pour la prototrophie pour la biotine en même temps (milieu LB + ampicilline 100 µg/ml + avidine 0,2 U/ml).

Les résultats observés sont rassemblés dans le tableau 1 :

## Tableau 1

| Génotype de la souche de E. coli | Sélection Amp. Transformants/µg d'ADN | Sélection sur milieu Amp + avidine 0,2 U/ml transformants/µg d'ADN |
|---|---|---|
| C268* Δ bioA, his | $> 10^3$ (pour chaque pool) | 3 (pool n° 4) 1 (pool n° 1) |
| C173* Δ bioD, his | $> 10^3$ (pour chaque pool) | 2 (pool n° 4) |

* : Cleary et Campbell (1972) J. Bacteriol. $\underline{112}$, 830.

Les plasmides sont isolés à partir des clones sélectionnés sur ampicilline + avidine et analysés en utilisant des enzymes de restriction. Trois plasmides (2 provenant de la souche C268 et 1 de la souche C173) contiennent un insert HindIII de 4,3 kb avec un site BglII et 2 sites SphI et sans site BamHI, SalI, PstI, EcoRV, PvuII, AvaI.

Avec l'un de ces plasmides, dénommé pTG1400, et dont la carte de restriction est représentée à la figure 2, il est possible de retransformer en sélectionnant pour la résistance à l'ampicilline et la prototrophie pour la biotine, aussi bien les souches C268 (ΔbioA), C173 (ΔbioD), R877 (bioD19) (Cleary et Campbell, 1972) ou C162 (bioB) avec une fréquence moyenne correspondant à celle obtenue pour la sélection avec l'ampicilline seule (plus de $10^3$ par µg d'ADN). Aucune complémentation de l'auxotrophie pour la biotine des souches R878 (bioC23) ou R901 (ΔbioA-D) (Cleary et Campbell, 1972) ne peut être obtenue en utilisant pTG1400.

b) Dans Bacillus subtilis

La complémentation des mutants bio de Bacillus subtilis pourrait se révéler difficile car il est connu que des délétions peuvent se produire avec une fréquence très importante dans les inserts étrangers clonés dans les plasmides réplicatifs usuels de Bacillus subtilis. C'est pourquoi il a été développé une nouvelle stratégie basée sur des essais de complémentation à l'aide d'un plasmide non réplicatif. L'intégration de plasmides non réplicatifs dans l'ADN génomique de Bacillus subtilis intervient avec une fréquence assez élevée (environ $10^4$ transformants/μg d'ADN), en utilisant des cellules naturellement compétentes de Bacillus subtilis, si des régions homologues existent entre l'ADN génomique et le plasmide.

La première étape consiste à intégrer le plasmide pTG475 dont la structure est indiquée à la figure 3 dans différents mutants bio de Bacillus subtilis.

Le plasmide pTG475 contient le gène XylE qui code pour l'enzyme $C_{2,3}$ oxygénase (Zukowski et al. (1983) PNAS USA, 80, 1101-1105), qui peut être utilisé comme marqueur chromogénique (jaune) et qui est exprimé sous le contrôle du promoteur inductible du gène de la levane sucrase de Bacillus subtilis. Ce plasmide comporte également un gène CAT conférant la résistance au chloramphénicol ; les gènes CAT et XylE sont insérés dans pBR322.

Ce plasmide est intégré dans le chromosome des souches suivantes de Bacillus subtilis :
. souche bioA : JKB 3173 (bioA 173, aro G932 ; CH Pai (1975) J. Bacteriol. 121, 1-8),
. souche bioB : BGSClA92 (bioB 141, aro G932 Sac A 321, Arg A2 ; CH Pai (1975) J. Bacteriol. 121, 1-8,
. souche bio112 : JKB 3112 (bio 112 ; CH Pai (1975) J. Bacteriol. 121, 1-8).
par la technique de transformation des cellules compétentes de R.J. Boyland (1972) J. Bacteriol. 110, 281-290, la sélection étant effectuée sur TBAB (DIFCO Blood tryptose agar base)) plus chloramphénicol 3 μg/ml.

Différents contrôles sont effectués sur les clones transformés : ils virent au jaune lorsqu'on induit avec le sucrose, et en outre un contrôle par hybridation Southern pour les souches bioA, bioB et bio112 montre que l'intégration de pTG475 par recombinaison simple dans le promoteur du gène de la levane sucrase a eu lieu. Ces souches transformées de Bacillus subtilis sont appelées bioA TG1, bioB TG2 et bio112 TG3. Le plasmide pTG475 ayant apporté des séquences de pBR322 dans le génome de Bacillus subtilis, il devient possible d'intégrer, par recombinaison homologue, tout plasmide étranger comportant ces mêmes séquences.

Dans une deuxième étape, le plasmide pTG1400, préalablement cloné par complémentation dans E. coli, a été utilisé pour transformer les souches de Bacillus subtilis bioA TG1, bioB TG2 et bio112 TG3. La sélection est effectuée sur milieu LB + avidine 0,2 U/ml + chloramphénicol 10 μg/ml.

On observe qu'il est possible de sélectionner, à une très haute fréquence, des transformants prototrophes pour la biotine à partir de souches bioA TG1 et bioB TG2 mais pas avec la souche bio112 TG3. pTG1400 ne complémente donc pas la mutation bio112.

## c) Caractérisation finale de l'insert HindIII de pTG1400

Des expériences d'hybridation Southern ont été effectuées de façon à détecter le même fragment HindIII dans l'ADN génomique de Bacillus sphaericus IFO 3525, correspondant à l'insert de pTG1400.

Dans des conditions d'hybridation drastiques (50 % de formamide, 0,6 % de solution de Denhardt, 0,1 % SDS, 3xSSC à 42°C) et en utilisant un plasmide pTG1400 marqué au $^{32}$P par incorporation de nucléotides radioactifs par polymérisation in vitro ( "nick translation") ($2,5.10^7$ cpm/μg d'ADN), une seule bande HindIII de 4,3 kb a pu être visualisée dans l'ADN génomique de Bacillus sphaericus IFO 3525 après 6 heures d'autoradiographie à -80°C. Il a été vérifié que dans ces conditions d'hybridation pBR322 ne donnait pas de réaction croisée avec l'ADN génomique de Bacillus sphaericus IFO 3525. Dans les mêmes conditions, aucune réaction positive, avec pTG1400 comme sonde, n'a pu être détectée dans l'ADN génomique de Bacillus subtilis BGSC1A289 traité par HindIII ou dans l'ADN génomique de E. coli C600 traité avec HindIII.

La séquence d'ADN totale du fragment HindIII de 4,3 kb a été analysée en utilisant la méthode "Shotgun", c'est-à-dire le clonage systématique des fragments obtenus par sonication, les "délétions cyclones" ou les élongations avec oligonucléotides-primers.

Pour la méthode "Shotgun", le plasmide pTG1400 a été découpé par traitement aux ultrasons. Après traitement des segments d'ADN avec l'ADN polymérase du phage T$_4$, les fragments aux extrémités franches sont mis à migrer sur un gel d'agarose à bas point de fusion et des fragments d'environ 300 pb sont isolés.

Le clonage de ces fragments a été réalisé dans des vecteurs M13 digérés par SmaI et traités à la phosphatase, .

Les plages blanches qui ne donnent pas d'hybridation croisée avec PBR322 sont triées et 100 clones sont séquencés. Les résultats ont été analysés par ordinateur.

Un procédé récent pour l'obtention d'une série de clones se chevauchant (le système cyclone) à été utilisé pour le séquençage de l'ADN (R.M.K. Dale, B.A. Mc Clure, J.P. Houchins (1985) Plasmid 13, 31-40). Cette méthode a été poursuivie en parallèle avec la méthode "Shotgun", de façon à confirmer les résultats. En outre, cette méthode produit des plasmides délétés définis contenant des groupes de gènes bio ou des gènes bio isolés.

La séquence complète du fragment HindIII de pTG1400 est représentée aux figures 4, 5, 6, 7 et 8.

L'analyse par ordinateur de cette séquence révèle que le fragment a la capacité de coder pour quatre longs cadres de lecture ouverts (LORF). Les sites possibles d'initiation de la traduction et les régions Shine et Dalgarno sont soulignées. Une région palindromique est soulignée à l'extrémité 3' de la séquence qui pourrait représenter un site de terminaison de transcription.

L'analyse de complémentation détaillée montre que la première région LORF (figure 5) (avec 3 sites d'initiation de traduction possibles) correspond au gène bioD.

Des expériences connues sous le nom de "maxicellules" ont été réalisées avec la souche de E. coli CSR 603 (recA1, phr-1, uvrA6, thr-1, leu-6, thi-1, argE3, lacY1, galK2, ara14, xyl15, mt11, proA2, str-31, tsx-33, supE44, F−, lambda−) ; elles montrent que cette région code pour un polypeptide avec d'un poids moléculaire apparent d'environ 25 kd.

La seconde région LORF (figure 6) (avec 4 sites d'initiation de traduction possibles) correspond au gène bioA. Une expérience en "maxicellules" de E. coli CSR603 révèle un polypeptide d'un poids moléculaire apparent d'environ 40 kd qui correspond au produit du gène bioA.

Il n'a pas été possible de déterminer la fonction de la troisième séquence LORF, appelée gène Y (figure 7). Une très grande hydrophobicité et la présence d'une séquence signal probable dans la région codante suggèrent que ce LORF, si il est transcrit et traduit, code pour une protéine ayant une interaction avec la membrane. Le fait que ce gène Y soit en "cluster" avec les autres gènes bio (A, D et B) suggère qu'il code pour une autre fonction impliquée dans le métabolisme de la biotine.

La quatrième région LORF (figure 8) (avec 3 sites d'initiation de la traduction possibles) a déjà été identifiée, il s'agit d'une région codant pour le gène bioB. Il est démontré ici que ce gène est lié en "cluster" avec les gènes bioA et bioD.

Une analyse de complémentation avec des plasmides contenant des régions sous-clonées de l'insert HindIII de 4,3 kb de pTG1400 montre clairement, comme cela est représenté à la figure 9, que la première region LORF correspond au produit du gèneD et que la seconde région LORF correspond au produit du gène A.

EXEMPLE 2

Clonage par complémentation du gène bioF de Bacillus sphaericus IFO 3525

La banque d'ADN génomique HindIII de Bacillus sphaericus IFO 3525 décrite précédemment a été utilisée pour transformer un mutant de E. coli, bioF 12, en suivant la méthodologie décrite précédemment.

Les résultats obtenus sont rassemblés au tableau 2 :

## Tableau 2

| Génotype de la souche de E. coli | Sélection Amp. | Sélection sur milieu Amp.+ avidine 0,2 U/ml |
|---|---|---|
| | Transformants/µg d'ADN | Transformants/µg d'ADN |
| R874* bioF 12, his | $> 10^4$ (pour chaque pool) | 2 (pool n° 1) |
| | | 2 (pool n° 3) |
| | | 4 (pool n° 4) |

\* Cleary et Campbell (1972) J. Bacteriol. 112, 830

Les plasmides sont isolés à partir des clones sélectionnés sur milieu contenant de l'ampicilline et de l'avidine et analysés en utilisant des enzymes de restriction.

Deux de ces plasmides contiennent deux inserts HindIII d'environ 4,5 et 0,6 kb avec des sites SphI, KpnI, HpaI, NdeI, AvaI, ClaI, BgI, XmnI, PvuII, ScaI, StuI et sans site BglII, XbaI, SmaI, PstI, SalI, NruI, BamHI, PvuI, EcoRI, HindII. EcoRV, FspI, ApaI, BalI, AatII..

Avec l'un de ces plasmides, pTG1418, il a été possible de retransformer en sélectionnant pour la résistance à l'ampicilline et la prototrophie pour la biotine, la souche R874 de E. coli (bioF12, his) avec une fréquence moyenne correspondant à celle obtenue pour la sélection des clones sur ampicilline (plus de 10⁴/µg d'ADN).

Des expériences de sous-clonage ont montré que seul le fragment HindIII de 4,5 kb code pour la fonction KAPA synthétase assurant la complémentation de la mutation bioF12 de la souche R874 de E. coli.

Des expériences de "Southern" ont été effectuées de façon à détecter le fragment HindIII de 4,5 kb dans l'ADN génomique de Bacillus sphaericus IFO 3525 en comparaison avec l'insert de 4,5 kb de pTG1418. En utilisant des conditions d'hybridation très drastiques (50 % de formamide, 3xSSC, 0,1 % SDS, 0,6 % de solution de Denhardt, 42° C) et en utilisant M13TG1425 (phage M13 contenant l'insert HindIII de 4,5 kb marqué au 32P par incorporation de nucléotides radioactifs par polymérisation in vitro ("nick translation)" avec 2.10⁶ cpm/µg d'ADN), une bande HindIII d'environ 4,5 kb peut être visualisée dans l'ADN génomique de Bacillus sphaericus IFO 3525 après 7 heures d'autoradiographie à -80°C.

Dans les mêmes conditions, aucune réaction positive ne peut être détectée, ni sur l'ADN génomique de Bacillus subtilis BGSC1A289 traité par HindIII ni sur l'ADN génomique de E. coli C600 traité par HindIII.

Aucune réaction croisée ne peut être détectée entre l' insert de pTG1400 et les inserts de pTG1418, ni avec un fragment SphI de 8,3 kb de pTG1406 chevauchant l'extrémité 3' de pTG1400, ni avec un fragment MboI de 8,2 kb de pSBO1 chevauchant l'extrémité 5' de pTG1400 (voir figure 10).

La carte de restriction des inserts de pTG1418 a été analysée et est indiquée à la figure 11.

Les études de complémentation et l'analyse "Southern" démontrent que le gène bioF de Bacillus sphaericus IFO 3525 n'est pas lié aux gènes bioA, bioD et bioB du même microorganisme (figure 12).

### EXEMPLE 3

Complémentation du mutant de B. subtilis bio112 TG3 (dérivé de JKB 3112, bioC/F-112 aroG932) par transformation intégrative avec pTG1418 et différents dérivés de celui-ci

Le mutant bio112 de B. subtilis a été identifié par des tests nutritionnels comme affecté dans la fonction bioF ou bioC (C.H. Pai, 1975, J. Bacterial 121, 1-8). Dans ce mutant, le plasmide pTG475 a été intégré au niveau du locus sacR - sacB, suivant la méthodologie décrite précédemment, la nouvelle souche ainsi obtenue a été dénommée bio112 TG3.

La transformation de la souche bio112 TG3 par différents plasmides (pTG1418, 1420, 1422, 1435, 1436 et 1437, représentés dans les figures 13 à 16) a été effectuée, suivie d'une sélection sur milieu LB + chloramphénicol 10 µg/ml + avidine 500 U/l. Les résultats de complémentation sont résumés dans le tableau 3. Comme le test croisé avec le mutant R874 (bioF, his) de E. coli donne le même résultat, il est vraisemblable que le mutant bio112 de B. subtilis est affecté dans le gène bioF. En analysant les complémentations obtenues en fonction des plasmides utilisés, il est possible de localiser le gène bioF sur l'insert de pTG1418 au niveau d'un fragment délimité par les sites de restriction XmnI et NcoI (voir figures 21 à 23).

## Tableau 3

## Transformation intégrative de la souche de
## B. subtilis bio112 TG3

| Plasmide | Insert | Complémentation sur LB + 500 U/l avidine + 10 µg/ml chloramphénicol |
|---|---|---|
| pTG1418 | Insert HindIII de 5,1 kb | ++ |
| pTG1422 | Insert ClaI de 3,1 kb contenant bioF et une partie de LORF W | ++ |
| TG1420 | Insert ClaI-HindIII de 2 kb contenant X et une partie de LORF W | - |
| pTG1436 | Insert XmnI-NcoI de 1,3 kb contenant le gène bioF | ++ |
| pTG1437 | Insert NcoI-XmnI de 1,3 kb contenant le gène bioF | ++ |
| pTG1435 | Insert HpaI-PvuII de 0,6 kb contenant le LORF X | - |

## EXEMPLE 4

### Séquence nucléotidique de l'insert HindIII de 4,53 kb du plasmide pTG1418

L'insert HindIII a été cloné dans le M13TG131 au niveau du site correspondant du polylinker. La méthode dite "cyclone" a été appliquée sur ce plasmide M13TG1425 et les résultats analysés par ordinateur. Les quelques différences de lecture entre les deux brins complémentaires ont été éclaircies sur séquençage à l'aide d'oligonucléotides spécifiques.

La séquence est détaillée dans les figures 17 à 19. Il apparaît que cet insert comporte trois cadres ouverts de lecture, codant respectivement pour des protéines (sous-unités) d'un poids moléculaire de 18462, 28048 et 42940 daltons. Le dernier gène, par rapport au sens de lecture, se trouve localisé dans la région identifiée comme bioF, le poids moléculaire de cette protéine de B. sphericus étant du même ordre de grandeur que celui de la KAPA synthétase de E. coli (M.A. Eisenberg, 1973, adv. Enzymol. 38, 317-372).

La juxtaposition des trois cadres ouverts de lecture pourrait être, comme dans le cas de l'insert du plasmide pTG1400, typique d'une structure opéronique. Il est à noter que l'on peut identifier, en amont du premier gène, une séquence de 15 paires de bases (soulignée dans la figure 17) présente également en amont du premier gène (bioD) de l'insert du plasmide pTG1400. Cette caractéristique significative pourrait indiquer que les deux groupes de gènes biotine de B. sphaericus sont soumis au moins à une régulation commune. Celle-ci pourrait correspondre au contrôle par la biotine (ou un dérivé de celle-ci), comme cela a déjà été décrit pour la KAPA synthétase (bioF) de B. sphaericus (Y. Yzumi, K. Sato, Y. Tani et K. Ogata, 1973, Agric. Biol. Chem. 37, 1335).

Du côté 3′ du dernier gène de l'insert séquencé, une séquence présentant les caractéristiques d'un terminateur de transcription peut être identifiée (soulignée dans la figure 18).

## EXEMPLE 5

### Complémentation des mutants E. coli R878 (bioC, his) et C261 (ΔbioFCD, his) respectivement, à l'aide des plasmides pTG1418 (1433) et pTG1440

La méthodologie traditionnelle des complémentations de mutants bio de E. coli a été appliquée en utilisant le plasmide pTG1418 et différents dérivés de celui-ci.

Lorsque des cellules compétentes du mutant E. coli R878 (bioC, his) sont transformées avec les plasmides pTG1418 et pTG1433 (voir tableau 4), puis ensuite étalées sur milieu LB + ampicilline 100 µg/ml + avidine 200 U/l, une croissance est détectée après 36 h d'incubation à 37°C. La fréquence d'apparition des clones transformés sur ce milieu est du même ordre de grandeur que celle mesurée sur le milieu LB + ampicilline 100 µg/ml.

### Tableau 4

### Transformation du mutant E. coli R878 bioC

### Nombre de transformants par µg d'ADN

| Plasmide | Sélection sur milieu LB + ampicilline (100 µg/ml) | Sélection sur milieu LB + ampicilline + avidine (100 µg/ml) (200 U/l) |
|---|---|---|
| pTG1418 | $10^3$ | $10^3$ petits |
| pTG1433 | $10^3$ | $10^3$ petits |
| pBR322 | $10^3$ | 0 |

(après 36 h d'incubation à 37°C)

Les croissances des clones transformés, normales sur LB + ampicilline, sont ralenties en l'absence de biotine (milieu LB + ampicilline + avidine ; minimum plus casaminoacides, dépourvu de biotine). Cette complémentation de l'auxotrophie en biotine du mutant R878 bioC est tout à fait significative, étant donné l'absence totale de croissance rédiduelle de ce même mutant, lorsqu'il est transformé par divers plasmides dérivés de pBR322, sur milieu dépourvu de biotine.

Les deux inserts des plasmides pTG1400 et pTG1418 ont été clonés dans pBR322 pour donner le plasmide pTG1440 (figure 20). Ce plasmide pTG1440, lorsqu'il est introduit dans le mutant E. coli C261 (ΔbioFCD, his) permet la sélection de clones sur milieu LB + ampicilline 100 µg/ml + avidine 200 U/l. La fréquence de transformation obtenue est directement comparable à celle mesurée sur LB + ampicilline. A nouveau, la croissance de ces clones recombinants, noramle sur milieu LB + ampicilline, est ralentie en l'absence de biotine. De ces deux résultats (complémentation de l'auxotrophie en biotine du mutant bioC et bioΔFCD), il ressort clairement que l'insert du plasmide pTG1418 contient également le gène bioC de B. sphaericus. Les différents sous-clonages dérivés de l'insert de pTG1418 (figures 21 à 23) ne permettent pas d'obtenir la complémentation de la mutation bioC de E. coli. Seuls les inserts possédant les trois gènes confèrent une complémentation effective de la mutation bioC de E. coli.

## EXEMPLE 6

### Complémentation du mutant E. coli bioH (PA505 MAΔ108, argH, metA, bioH, malA, strr)

Ce mutant a été décrit à l'origine comme bioB (D. Hatfield, M. Hofnung et M. Schwartz, 1969, J. Bacteriol. 98? 559-567). Il a été ensuite caractérisé comme n'excrétant aucun vitamère et capable de croître sur milieu minéral en présence de KAPA, DAPA, DTB ou biotine. Eisenberg (1985, Annals New York Academy of Sciences 447, 335-349) a ensuite proposé que ce gène code pour une sous-unité de la pimeloyl-CoA-synthétase (bioH). Il est à noter que les mutants de E. coli surproducteurs de biotine (sélectionnés soit par un niveau d'excrétion de vitamine permettant la croissance d'un auxotrophe bioB de E. coli, soit par résistance à l'alpha-déhydrobiotine) ont tous été identifiés génétiquement comme affectés au locus bioR. Ce locus code pour une protéine multifonctionnelle (répresseur de la synthèse des ARN messagers de l'opéron bioABFCD et holoenzyme synthétase fixant la biotine sur un résidu lysine de différentes apoenzymes à fonction carboxylase).

Le fait que tous les mutants de E. coli surproducteurs de la biotine identifiés à ce jour sont localisés dans le gène codant pour le répresseur actif en trans et jamais dans l'opérateur de l'opéron bioABFCD laisse supposer qu'un autre gène de la biosynthèse de la biotine est soumis à cette régulation. Au regard de la littérature, le meilleur candidat est le gène bioH.

L'insert de pTG1418 contenant les gènes bioF et bioC de B. sphaericus, on a recherché si le troisième gène correspondait à bioH.

La complémentation du mutant bioH de E. coli a effectivement été obtenue en utilisant le plasmide pTG1433. Une nouvelle fois, la croissance obtenue sur ce milieu est ralentie, mais tout à fait significative par rapport aux témoins (voir tableau 5).

### Tableau 5

### Transformation du mutant E. coli bioH PA505 MAΔ108

### Nombre de transformations par µg d'ADN

| Plasmide | Sélection sur milieu LB + ampicilline (100 µg/ml) | Sélection sur milieu LB + ampicilline + avidine (100 µg/ml)    (200 U/l) |
|---|---|---|
| pBR322 | $10^4$ | 0 |
| pTG1433 | $10^3$ | $10^3$ petits |
|  |  | (après 36 h d'incubatior à 37°C) |

De même que pour la complémentation du mutant bioC, une condition nécessaire et suffisante pour la complémentation du mutant bioH est la présence simultanée des trois gènes de l'insert de pTG1418 sur les plasmides introduits dans la souche (figures 21 à 23).

Au contraire des mutants bioF de E. coli et de B. subtilis, pouvant être complémentés par un seul gène de B. sphaericus, la croissance en l'absence de biotine des mutants bioC et bioH de E. coli ne peut donc être obtenue que lors de l'introduction des plasmides recombinants porteurs des trois gènes de l'insert HindIII du pTG1418. Ceci pourrait refléter, entre autres, des différences de propriétés enzymatiques entre la piméloyl-CoA-synthétase de B. sphaericus et l'enzyme correspondante de E. coli (différence d'affinité vis-à-vis des substrats, édifice multienzymatique, notamment) ou une synthèse réduite de la piméloyl-CoA-synthétase de B. sphaericus dans E. coli, limitant le flux métabolique du pimélate vers le KAPA.

## EXEMPLE 7

### Test fonctionnel des gènes bio FCH

Les tests de complémentation des mutants bioF, bioH et bioC de E. coli à l'aide de plasmides recombinants porteurs d'inserts dérivant du fragment HindIII de 4,5 kb isolé à partir de B. sphaericus peuvent être reconnus comme une preuve de la présence sur cet ADN des gènes bioF, bioH et bioC.

Néanmoins, il est utile de compléter cette information par un test d'activité des produits de ces gènes clonés à partir de B. sphaericus. Le produit du gène bioF de E. coli a été caractérisé comme catalysant la transformation du piméloyl-CoA- en KAPA (Eisenberg, 1968). Les produits des deux gènes bioC et bioH ne sont pas clairement identifiés à l'heure actuelle ; une hypothèse citée dans la littérature (Eisenberg, 1985) suggère qu'ils codent pour les protéines impliquées dans la biosynthèse du piméloyl-CoA. On a testé si la séquence bioF, C, H assure spécifiquement la transformation du pimélate en KAPA.

L'insert porteur de la partie codante FCH de B. sphaericus (récupéré sous forme du fragment EcoRV-SphI du pTG1434 associé au fragment SphI-SphI du pTG1436, ligués ensemble) a été fusionné au promoteur du gène conférant la résistance à la tétracycline de pBR322 pour donner le plasmide pTG1446, afin d'obtenir un niveau d'expression significatif des protéines associées aux gènes bioF, C, H.

Ce plasmide a ensuite été introduit dans des cellules compétentes de la souche bioH de E. coli. Cette souche recombinante est ensuite mise en culture à 37°C pendant 48 heures dans le milieu GP (pour 1 litre : glycérol : 20 g ; protéose peptone : 30 g ; casamino acids vitamine free : 5 g ; $K_2HOP_4$ : 1 g ; KCl : 0,5 g ; $MgSO_4-7H_2O$ : 0,5 g ; $FeSO_4-7H_2O$ : 0,01 g ; $MnSO_4-4H_2O$ : 0,001 g pH 6,8-7 ; Thiamine-HCl : 20 µg) additionné d'ampicilline 100 µG/ml et de pimélate (pH 7,5) 0,5 mg/ml.

Une aliquote du surnageant (5 µl) est ensuite mise en chromatographie sur plaque de silice (solvant chromato : n butanol [60] -acide acétique [15] - eau [25], vol/vol, migration du solvant : 10 cm) pour séparer les vitamères et la biotine produits.

Après migration spécifique à chaque vitamère, le KAPA est évalué quantitativement par dosage biologique en utilisant une souche de Saccharomyces cerevisiae de collection : ATCC 7754.

Dans ces conditions, on peut mesurer de manière reproductible une quantité de 85 µg (exprimée en équivalents biotine) de KAPA par ml de surnageant de culture.

Le témoin dans cette expérience est la même souche contenant le plasmide pBR322 mise en culture dans les mêmes conditions. Dans ce cas, aucune détection significative de KAPA ne peut être mise en évidence.

Il est donc démontré ici que l'insert du pTG1418 code pour les fonctions enzymatiques nécessaires et suffisantes dans la transformation du pimélate en KAPA, soit les produits des gènes bioC, H et F.

### Dépôt des souches représentatives de l'invention

Les souches de E. coli C600 pTG1400 et R874 pTG1418 ont été déposées à la Collection Nationale de Cultures de Microorganismes de l'Institut Pasteur, 28 rue du Docteur-Roux - 75724 Paris Cédex 15, le 26 septembre 1986 sous les n° I-608 et I-609.

REFERENCES

P.P. Cleary et A. Campbell (1972) J. Bacteriol. 112, 830-839.
M.M. Zukowski, D.F. Gaffney, D. Speck, M. Kauffmann, A. Findeli, A. Wisecup et J.P. Lecocq (1983) PNAS USA 80, 1101-1105.
C.H. Pai (1975) J. Bacteriol. 121, 1-8.
R.J. Boyland, N.H. Mendelson, D. Brooks et F.E. Young (1972) J. Bacteriol. 110, 281-290.
R.M.K. Dale, B.A. Mc Clure, J.P. Houchins (1986) Plasmid 13, 31-40.
S.N. Cohen, A.C.Y. Cheng, L. Hsu (1972) PNAS USA 69, 2110-2114.
H. Saito et K.I. Miura (1963) BBA 72, 619-629.
M.A. Eisenberg (1985) Regulation of the biotin operon. Annals New York Academy of Sciences, 447, 335-349.
M.A. Eisenberg et C. Star (1968) J. Bacteriol., 96, 1291-1297.

## Revendications

1) Séquence d'ADN codant pour l'enzyme produit de l'un des gènes suivants de la chaîne de biosynthèse de la biotine chez les bactéries:
. gène bioA
. gène bioD
. gène bioF
. gène bioC
. gène bioH

2) Séquence d'ADN selon la revendication 1, caractérisée en ce qu'elle code pour les enzymes produits des gènes bioB et bioD.

3) Séquence d'ADN selon la revendication 1, caractérisée en ce qu'elle code pour les enzymes produits des gènes bioD, bioA et bioB.

4) Séquence d'ADN selon la revendication 1, caractérisée en ce qu'elle code pour les enzymes produits des gènes bioD, bioA, bioB et bioF.

5) Séquence d'ADN codant pour l'enzyme produit de l'un des gènes suivants de la chaîne de biosynthèse de la biotine chez les bactéries:
. bioF et bioC
. bioF et bioH
. bioF, bioC et bioH.

6) Séquence d'ADN selon la revendication 5, caractérisée en ce qu'elle code, en outre, pour au moins l'enzyme produit de l'un des gènes suivants :
. bioA
. bioA et bioD.

7. Séquence d'ADN selon l'une des revendications 5 ou 6, caractérisée en ce qu'elle code pour les enzymes produits des gènes bioD, bioA et bioB.

8) Séquence d'ADN selon l'une des revendications 1 à 7, caractérisée en ce qu'elle est dépourvue des séquences naturelles assurant le contrôle de la transcription des enzymes de la voie de biosynthèse de la biotine chez la bactérie d'origine.

9) Séquence d'ADN en ce qu'elle correspond à tout ou partie de la séquence décrite dans les figures 4 à 8 et 17 à 19.

10) Séquence d'ADN selon l'une des revendications 1 à 9, caractérisée en ce qu'elle provient d'une souche de Bacillus.

11) Séquence d'ADN selon la revendication 10, caractérisée en ce qu'elle provient d'une souche de Bacillus sphaericus.

12) Vecteur plasmidique comportant au moins une séquence selon l'une des revendications 1 à 11.

13) Vecteur selon la revendication 12, caractérisé en ce qu'il s'agit d'un vecteur qui comporte les séquences d'ADN permettant son intégration dans un chromosome dans la souche hôte.

14) Vecteur selon la revendication 13, caractérisé en ce qu'il comporte au moins une séquence homologue d'une séquence présente dans le génome de la souche à transformer et assurant l'intégration.

15) Vecteur selon la revendication 14, caractérisé en ce que la séquence homologue est une séquence génomique naturelle.

16) Vecteur selon la revendication 14, caractérisé en ce que la séquence homologue est une séquence qui a été intégrée dans le génome grâce à un vecteur plasmidique d'intégration.

17) Vecteur selon la revendication 16, caractérisé en ce que le vecteur plasmidique spécifique comporte au moins un gène de résistance à un antibiotique et un gène marquer sous la dépendance d'un promoteur de la souche à transformer.

18) Vecteur selon la revendication 17, caractérisé en ce que le promoteur est un promoteur inductible.

19) Vecteur selon la revendication 18, caractérisé en ce que le vecteur plasmidique d'intégration est le plasmide pTG475.

20) Vecteur selon la revendication 12, caractérisé en ce qu'il comporte une origine de réplication autonome.

21) Vecteur selon la revendication 20, caractérisé en ce qu'il s'agit d'une origine de réplication autonome dans une souche de Bacillus ou une souche d'Escherichia.

22) Vecteur selon l'une des revendications 20 et 21, caractérisé en ce que les séquences d'ADN codant pour les enzymes de la chaîne de biosynthèse de la biotine sont flanquées d'éléments assurant leur expression dans la souche hôte.

23) Vecteur selon la revendication 22, caractérisé en ce que parmi les éléments assurant leur expression figure un promoteur et un terminateur efficaces dans la souche hôte.

24) Cellules transformées par un vecteur selon l'une des revendications 12 à 23.

25) Cellule selon la revendication 24, caractérisée en ce qu'il s'agit d'une bactérie choisie parmi les genres Bacillus, Escherichia ou Pseudomonas.

26) Cellule selon la revendication 24, caractérisée en ce qu'il s'agit d'une levure du genre Saccharomyces.

27) Cellule selon la revendication 25, caractérisée en ce que la bactérie est choisie parmi Bacillus sphaericus, Bacillus subtilis et Escherichia coli.

28) Cellule selon l'une des revendications 24 à 27, caractérisée en ce que la cellule transformée est une cellule productrice de biotine ou de l'un de ses vitamères.

29) Procédé de préparation de biotine caractérisé en ce qu'on fait fermenter un milieu de culture contenant au moins de l'acide pimélique ou l'un des vitamères de la biotine par des cellules selon l'une des revendications 24 à 28, perméables à l'acide pimélique ou au dit vitamère de la biotine et en ce qu'on récupère la biotine formée.

30) Procédé selon la revendication 29 caractérisé en ce que le vitamère de la biotine est obtenu par fermentation d'un milieu de culture contenant au moins de l'acide pimélique par une cellule selon l'une des revendications 24 à 28 produisant ledit vitamère.

31) Procédé selon la revendication 30, caractérisé en ce qu'on effectue une co-fermentation de deux souches de cellules selon l'une des revendications 24 à 28, l'une produisant le vitamère de la biotine à partir de l'acide pimélique et l'autre fermentant ce vitamère pour produire de la biotine.

PIMELATE (P.M. 160)

0266240

FIG _1

CoASH
ATP (MgCl₂)

bio C

pimelyl – CoA synthetase

PIMELYL - CoA

L-alanine
PLP

bioF

7 - KAPA synthetase

7 - KAPA

(acide 7-ceto - 8-aminopélargonique)

Sam
PLP

bio A

DAPA- aminotransférase

DAPA

(acide 7,8 diaminopélargonique)

HCO₃⁻
ATP(MgCl₂)

bio D

DTB synthétase

DTB

(d – desthiobiotine ) ( P.M. 214)

bio B    biotine synthétase

d – BIOTINE (P.M. 244)

pTG 1400
8740 bp

pBR 322 DNA

B. SPHAERICUS INSERT.

START BIO B

Pst I 7990
Pvu I 8116
Hind II 8288
Eco RI O
HindIII 29
Xmn I 41
Nru I 430
Hpa I - Hind II 561
Nde I 710

SAU3A-Mbo I
Nde I 1806
Dra III 1621
Hind II 1827
Nru I 2053
Bgl I 2131
Sph I 2150
Xba I 2215
Sph I 2282
Mst I 258
Bgl II 2724
Nde I 2819

Nde I 6678
Pvu II 6447

Nru I 5353
Sal I - Hind II 5032
Sph I 4943
Bam HI 4756
Eco RV 4566
Hind III 4407
Nde I 4195
Hpa I - Hind II 3923
Hpa I - Hind II 3945

FIG_2

0266240

FIG_3

# FIG.4

```
          10         20         30         40         50
AAGCTTTGCA CACTTCTGTT TCGTATCCTC ATATTGAACT TGATGAAACC

          60         70         80         90        100
TTCCTATGGC CGTATGCATT GAGATTTTTT CTCGATGTTC TGCTTGCAAT

         110        120        130        140        150
GTTCGATATT CTTCTTGCCG AATAGCTACA CGATACCAAA ATTCATAACG

         160        170        180        190        200
CAACGGTAAA TCTCTTATTT CGTAAGTAAG CAAAGTATTT AAAATACTGC

         210        220        230        240        250
TCATTTGTTC ATATGTATCT AGCTTTTTAT CTGTCTCCTT AAATAGTCCA

         260        270        280        290        300
AACATTTTGC CACCCCCTGT TTTGATTAAT ACTACAACCT ATGATAAAAA

         310        320        330        340        350
CCCTTTAATA TTTCTTGGGA AATAATCCAA CGTTGATAAA ACGGGGTGAA

         360        370        380        390        400
TATCCGATCA ATCGAGTGAA ATTTAGGATA GAATACCCTC GGAAAAAGCA

         410        420        430        440        450
TTATCTGAAT CATTTATGTA AAAATGCAAA AAAAGGCATT TACAAAAGGA

              460        470        480
          AAAAGAATGT GTTAACTTAA AAACTATAGT              TGGTT
```

# FIG. 5

AAAAAGAATGTGTTAACTTAAAAACTATAGTTGGT

**RBS**
_____

TAA CTA AAA GAG GGG GAG GTA CAG [TTG] CAA
*** Leu Lys Glu Gly Glu Val Gln Leu Gln

CAC TTT TGG GTT GTT GGA ACA GAT ACA GAT
His Phe Trp Val Val Gly Thr Asp Thr Asp

GTT GGA AAA ACA TTT GTC ACC ACA TTA TTA
Val Gly Lys Thr Phe Val Thr Thr Leu Leu

[ATG] CGT AAT [TTG] CAA AAA CAG GGC GTA CGT
Met Arg Asn Leu Gln Lys Gln Gly Val Arg

GTA ACG CCT TAT AAA CCA GTC CAA ACT GGT
Val Thr Pro Tyr Lys Pro Val Gln Thr Gly

GAA GTG TAT GAT GGT GAA CAA GCC TAT TAC
Glu Val Tyr Asp Gly Glu Gln Ala Tyr Tyr

TTC GAC ACA GCG ATG TAT GAA AAA TAT TCC
Phe Asp Thr Ala Met Tyr Glu Lys Tyr Ser

TTG CAA TTG CTA GAC AGA GAG AAT TTA AAT
Leu Gln Leu Leu Asp Arg Glu Asn Leu Asn

GGC TAT TCA TTT AAA GAG GCT GCA TCG CCA
Gly Tyr Ser Phe Lys Glu Ala Ala Ser Pro

CAT TTT GCG GCT CAA CTG GAG GGG CAG CAA
His Phe Ala Ala Gln Leu Glu Gly Gln Gln

# FIG. 5

785
ATT GAC ACA CAG CAG TTA TTA AAG CAA ATG
Ile Asp Thr Gln Gln Leu Leu Lys Gln Met

815
CAA CTT TTA CAG CAA ACA TGG GAT GTT GTT
Gln Leu Leu Gln Gln Thr Trp Asp Val Val

845
ATT TGT GAA GGA GCG GGT GGG CTC TTT GTG
Ile Cys Glu Gly Ala Gly Gly Leu Phe Val

875
CCA TTA GAT GCA TGT GGC GAA ACG ACA TTG
Pro Leu Asp Ala Cys Gly Glu Thr Thr Leu

905
TTG GAT GTC ATT GTT GAA AGT AAA CTA CCC
Leu Asp Val Ile Val Glu Ser Lys Leu Pro

935
GTT GTC GTG GTT ACA CGA ACA GCA CTA GGA
Val Val Val Val Thr Arg Thr Ala Leu Gly

965
ACA ATT AAC CAT ACG CTC TTA ACG TTA GAG
Thr Ile Asn His Thr Leu Leu Thr Leu Glu

995
GCA TTG ACT ACA CGG AAA ATT GAA GTG CTT
Ala Leu Thr Thr Arg Lys Ile Glu Val Leu

# FIG. 5

1025
GGT CTT GTA TTT AAC GGT GAT ATG GGG AGC
Gly Leu Val Phe Asn Gly Asp Met Gly Ser

1055
AGG ATG GAG CAA GAC AAT ATC CAA ACG ATT
Arg Met Glu Gln Asp Asn Ile Gln Thr Ile

1085
TTA CAG TAT TAT ACA TTG CCC TAT ATG ACG
Leu Gln Tyr Tyr Thr Leu Pro Tyr Met Thr

1115
ATA CCA AAG CTG GAA GAG CTG TCG GAC ATT
Ile Pro Lys Leu Glu Glu Leu Ser Asp Ile

1145
AAT GAG TAT GCA ATT ACG GGC ACA TCA TTG
Asn Glu Tyr Ala Ile Thr Gly Thr Ser Leu

1175
TTT GAA AGG CTG ATT AGA CGT GAA ACA AGT
Phe Glu Arg Leu Ile Arg Arg Glu Thr Ser

1205
ATT AAC TGA GCT ACA AGA
Ile Asn ***

1235
AAA AGA TTT ACA ACA TGT

GTATGCAATTACGGGCACATCATTGTTTGAAAGGC

1185
TGA TTA GAC GIG AAA CAA GTA TTA ACT GAG
*** Leu Asp Val Lys Gln Val Leu Thr Glu

1215
CTA CAA GAA AAA GAT TTA CAA CAT GTC TGG
Leu Gln Glu Lys Asp Leu Gln His Val Trp

1245
CAT CCT TGC TCA CAA ATG AAA GAT TAT GAG
His Pro Cys Ser Gln Met Lys Asp Tyr Glu

1275
GCT TTT CCA CCA ATC GTT ATA AAA AAA GGC
Ala Phe Pro Pro Ile Val Ile Lys Lys Gly

1305
GAA GGT GTA TGG CTG TAT GAT GAA CAG AAT
Glu Gly Val Trp Leu Tyr Asp Glu Gln Asn

1335
CAA CGC TAT CTT GAT GCG GTA TCT TCA TGG
Gln Arg Tyr Leu Asp Ala Val Ser Ser Trp

1365
TGG GTC AAT TTA TTT GGA CAT GCC AAT CCA
Trp Val Asn Leu Phe Gly His Ala Asn Pro

1395
CGT ATT AGC CAA GCA TTA AGT GAA CAA GCA
Arg Ile Ser Gln Ala Leu Ser Glu Gln Ala

1425
TTT ACG TTG GAG CAT ACA ATT TTT GCG AAT
Phe Thr Leu Glu His Thr Ile Phe Ala Asn

1455
TTT TCA CAT GAG CCA GCG ATT AAA CTC GCA
Phe Ser His Glu Pro Ala Ile Lys Leu Ala

1485
CAA AAA TTA GTA GCT TTA ACA CCA CAA AGT
Gln Lys Leu Val Ala Leu Thr Pro Gln Ser

```
1515
    TTA CAA AAA GTA TTT TTT GCA GAT AAT GGT
    Leu Gln Lys Val Phe Phe Ala Asp Asn Gly
1545
    TCA TCT GCT ATA GAA GTC GCT TTA AAA [ATG]
    Ser Ser Ala Ile Glu Val Ala Leu Lys Met
1575
    AGT TTT CAA TAT CAT ATG CAA ACG GGG AAA
    Ser Phe Gln Tyr His Met Gln Thr Gly Lys
1605
    ACG CAA AAA AAA CGC TTT TTG GCA TTA ACG
    Thr Gln Lys Lys Arg Phe Leu Ala Leu Thr
1635
    GAT GCC TAC CAT GGT GAA ACA TTA GGT GCT
    Asp Ala Tyr His Gly Glu Thr Leu Gly Ala
1665
    TTA TCC GTC GGT GGC GTA GAT CTT TAT AAC
    Leu Ser Val Gly Gly Val Asp Leu Tyr Asn
1695
    GAA GTG TAT CAA CCA CTG TTA TTG GAT ACG
    Glu Val Tyr Gln Pro Leu Leu Leu Asp Thr
1725
    GTA CGA GCA CAA GGC CCA GAT TGT TTC CGT
    Val Arg Ala Gln Gly Pro Asp Cys Phe Arg
1755
    TGC CCA TTC AAG CAT CAT CCG GAT AGT TGC
    Cys Pro Phe Lys His His Pro Asp Ser Cys
1785
    CAT GCC CAA TGT ATT AGT TTT GTA GAG GAT
    His Ala Gln Cys Ile Ser Phe Val Glu Asp
1815
    CAG TTG CGC ATG CAT CAT AAG GAA ATT ACG
    Gln Leu Arg Met His His Lys Glu Ile Thr
1845
    GCG GTT ATT ATT GAG CCA CTC ATT CAA GCG
    Ala Val Ile Ile Glu Pro Leu Ile Gln Ala
```

1875
```
GCA GCA GGG ATG AAA ATG TAT CCA GCT ATT
Ala Ala Gly Met Lys Met Tyr Pro Ala Ile
```

1905
```
TAT TTG CGA CGT TTA CGT GAA CTA TGT ACG
Tyr Leu Arg Arg Leu Arg Glu Leu Cys Thr
```

1935
```
CAA TAT GAT GTG CAT CTA ATT GCA GAC GAA
Gln Tyr Asp Val His Leu Ile Ala Asp Glu
```

1965
```
ATT GCT GTA GGT TTT GGG CGC ACA GGT ACA
Ile Ala Val Gly Phe Gly Arg Thr Gly Thr
```

1995
```
CTT TTT GCC TGT GAG CAG GCT AAT ATC TCT
Leu Phe Ala Cys Glu Gln Ala Asn Ile Ser
```

2025
```
CCG GAT TTT ATG TGT TTA TCA AAA GGT TTA
Pro Asp Phe Met Cys Leu Ser Lys Gly Leu
```

2055
```
ACA GGT GGG TAT TTA CCA CTG TCT GTC GTA
Thr Gly Gly Tyr Leu Pro Leu Ser Val Val
```

2085
```
ATG ACG ACG AAT GAT GTA TAT CAG GCA TTT
Met Thr Thr Asn Asp Val Tyr Gln Ala Phe
```

2115
```
TAT GAT GAT TAT GCC ACG ATG AAG GCG TTT
Tyr Asp Asp Tyr Ala Thr Met Lys Ala Phe
```

2145
```
TTA CAT TCA CAT AGT TAC ACA GGG AAT ACA
Leu His Ser His Ser Tyr Thr Gly Asn Thr
```

2175
```
CTT GCC TGC CGT GTT GCT CTA GAG GTA TTG
Leu Ala Cys Arg Val Ala Leu Glu Val Leu
```

2205
```
GCG ATA TTT GAA GAA GAA CAG TAT ATA GAC
Ala Ile Phe Glu Glu Glu Gln Tyr Ile Asp
```

## FIG.6

```
2235
    GTT GTG CAA GAC AAA GGT GAA CGC ATG CGA
    Val Val Gln Asp Lys Gly Glu Arg Met Arg

2265
    AAG CTA GCC TTG GAG GCT TTT AGT GAT TTA
    Lys Leu Ala Leu Glu Ala Phe Ser Asp Leu
2295
    CCT TTT GTT GGT GAA TAT CGG CAA GTT GGG
    Pro Phe Val Gly Glu Tyr Arg Gln Val Gly

2325
    TTT GTC GGG GCG ATT GAA CTT GTG GCG AAT
    Phe Val Gly Ala Ile Glu Leu Val Ala Asn

2355
    CGC GAT ACC AAA GAG CCA TTA CCG AGT GAG
    Arg Asp Thr Lys Glu Pro Leu Pro Ser Glu

2385
    GAG CGC ATC GGC TAT CAA ATA TAC AAA AGA
    Glu Arg Ile Gly Tyr Gln Ile Tyr Lys Arg

2415
    GCT TTA GCA AAA GGG TTA CTG ATT CGT CCA
    Ala Leu Ala Lys Gly Leu Leu Ile Arg Pro

2445
    CTT GGG AAT GTT TTG TAT TTC ATG CCA CCA
    Leu Gly Asn Val Leu Tyr Phe Met Pro Pro

2475
    TAC ATT ATA ACG GAC GAT GAA ATG CAA TTT
    Tyr Ile Ile Thr Asp Asp Glu Met Gln Phe

2505
    ATG ATT CAA ACA ACA AAA GAT ACA ATT GTT
    Met Ile Gln Thr Thr Lys Asp Thr Ile Val

2535
    CAA TTT TTT GAA GAG CGG GAG GGA TGA GGG
    Gln Phe Phe Glu Glu Arg Glu Gly ***

2565                                        2595
    CAT GTT GAA ACA ACA GTC AAC GTT ATC ACT TGT GAT
```

# FIG_7

TATAACGGACGATGAAATGCAATTTA

2506
TGA TTC AAA CAA CAA AAG ATA CAA TTG TTC
*** Phe Lys Gln Gln Lys Ile Gln Leu Phe

2536
AAT TTT TTG AAG AGC GGG AGG GAT GAG GGC
Asn Phe Leu Lys Ser Gly Arg Asp Glu Gly

2566
ATG TTG AAA CAA CAG TCA ACG TTA TCA CTT
Met Leu Lys Gln Gln Ser Thr Leu Ser Leu

2596
GTG ATG ATT GCG ATG TTT GCT GCA TTA ACA
Val Mat Ile Ala Met Phe Ala Ala Leu Thr

2626
GCA GTT GGT GCC TTC ATT AAA ATT CCA TTA
Ala Val Gly Ala Phe Ile Lys Ile Pro Leu

2656
CCG CTC GTG CCG TTT ACA TTA CAA ATT GTC
Pro Leu Val Pro Phe Thr Leu Gln Ile Val

2686
TTT GTC TTT TTA GCG GGT TGC TTA CTC GGT
Phe Val Phe Leu Ala Gly Cys Leu Leu Gly

2716
GGT CGC AAT GGA TTT CAA AGT CAG CTA GTT
Gly Arg Asn Gly Phe Gln Ser Gln Leu Val

2746
TAC ATA GGA ATA GGT TTA GTT GGC TTG CCA
Tyr Ile Gly Ile Gly Leu Val Gly Leu Pro

2776
GTT TTT ACA CAA GGT GGA GGC ATT ACA TAT
Val Phe Thr Gln Gly Gly Gly Ile Thr Tyr

2806
GTA TTG CAG CCG ACT TTT GGT TAC TTA ATA
Val Leu Gln Pro Thr Phe Gly Tyr Leu Ile

2836
GGA TTT GCT CTT GCT GCA TTA GTA ATC GGC
Gly Phe Ala Leu Ala Ala Leu Val Ile Gly

2866
TAT ATG ATT GAT CGA GTA GAA TCA CCA ACG
Tyr Met Ile Asp Arg Val Glu Ser Pro Thr

2896
AAA AAG CAT TTC ATT GTT GCC AAT ATT ATA
Lys Lys His Phe Ile Val Ala Asn Ile Ile

2926
GGG CTT ATC ATT ATT TAT GCA GTC GCA GTA
Gly Leu Ile Ile Ile Tyr Ala Val Ala Val

2956
CCT TAT TTA TAT GTA GCA TTA AAT GTA TGG
Pro Tyr Leu Tyr Val Ala Leu Asn Val Trp

2986
TTA AAC ATG AAA TCA AGT TGG TCT CAT GTA
Leu Asn Met Lys Ser Ser Trp Ser His Val

3016
TTT TTA GTA GGC TTT GTC AAT AGT ATT GTT
Phe Leu Val Gly Phe Val Asn Ser Ile Val

3046
GCA GAC TTT TGC TTA GCA ATT GCT TCT GCC
Ala Asp Phe Cys Leu Ala Ile Ala Ser Ala

3076
CTT TTA GCT GAA CGT CTA TAC AAA GTA TTC
Leu Leu Ala Glu Arg Leu Tyr Lys Val Phe

3106
CGT TCC GCT AGA GCT ATA AAA CTT GTG CAA
Arg Ser Ala Arg Ala Ile Lys Leu Val Gln

3136                                        3166
ATT GAA AAG GAG AAT GTT TAG TGA ATT GGT TAC AAT
Ile Glu Lys Glu Asn Val *** ***

3196
TAG CAG ATG   AAG TGA TTG CAG GCA AGG TA

0266240

## FIG.8

TATAAAACTTGTGCAAAT

**RBS**

3138

TGA AAA GGA GAA TGT TTA GTG AAT TGG TTA
\*\*\* Lys Gly Glu Cys Leu Val Asn Trp Leu

3168

CAA TTA GCA GAT GAA GTG ATT GCA GGC AAG
Gln Leu Ala Asp Glu Val Ile Ala Gly Lys

3198

GTA ATT AGC GAT GAT GAG GCA CTT GCC ATT
Val Ile Ser Asp Asp Glu Ala Leu Ala Ile

3228

TTA AAT AGT GAT GAT GAT GAT ATT TTA AAG
Leu Asn Ser Asp Asp Asp Asp Ile Leu Lys

3258

CTA ATG GAC GGC GCA TTT GCC ATT CGT AAG
Leu Met Asp Gly Ala Phe Ala Ile Arg Lys

3288

CAC TAT TAC GGT AAA AAA GTA AAG TTA AAT
His Tyr Tyr Gly Lys Lys Val Lys Leu Asn

3318

ATG ATT ATG AAT GCT AAA AGT GGC TAT TGC
Met Ile Met Asn Ala Lys Ser Gly Tyr Cys

3348

CCA GAG GAT TGT GGC TAT TGC TCG CAG TCA
Pro Glu Asp Cys Gly Tyr Cys Ser Gln Ser

3378

TCT AAA TCG ACC GCT CCT ATT GAG AAA TAT
Ser Lys Ser Thr Ala Pro Ile Glu Lys Tyr

3408

CCG TTC ATT ACA AAA GAA GAA ATA TTA GCG
Pro Phe Ile Thr Lys Glu Glu Ile Leu Ala

3438

GGG GCA AAG CGT GCG TTT GAA AAT AAA ATT
Gly Ala Lys Arg Ala Phe Glu Asn Lys Ile

FIG_8

```
3468
    GGT ACG TAT TGC ATC GTC GCA AGC GGA CGT
    Gly Thr Tyr Cys Ile Val Ala Ser Gly Arg

 3498
    GGG CCG ACT CGT AAA GAT GTC AAT GTA GTG
    Gly Pro Thr Arg Lys Asp Val Asn Val Val

3528
    AGT GAA GCC GTT GAA GAA ATT AAA GCA AAA
    Ser Glu Ala Val Glu Glu Ile Lys Ala Lys

3558
    TAT GGC TTA AAA GTT TGC GCT TGC TTA GGT
    Tyr Gly Leu Lys Val Cys Ala Cys Leu Gly

3588
    TTA CTA AAA GAA GAA CAA GCA CAA CAA TTA
    Leu Leu Lys Glu Glu Gln Ala Gln Gln Leu

3618
    AAA GAA GCG GGT GTT GAT CGC TAC AAT CAT
    Lys Glu Ala Gly Val Asp Arg Tyr Asn His

3648
    AAC TTA AAT ACA TCA GAG CGT CAC CAT TCC
    Asn Leu Asn Thr Ser Glu Arg His His Ser

 3678
    TAT ATT ACG ACG ACG CAG ACA TAT GAG GAT
    Tyr Ile Thr Thr Thr His Thr Tyr Glu Asp

3708
    CGT GTT AAT ACC GTT GAG GTT GTA AAG AAA
    Arg Val Asn Thr Val Glu Val Val Lys Lys

3738
    CAT GGT ATT TCC CCA TGT TCT GGA GCC ATT
    His Gly Ile Ser Pro Cys Ser Gly Ala Ile

3768
    ATT GGG ATG AAA GAA ACG AAA ATG GAT GTC
    Ile Gly Met Lys Glu Thr Lys Met Asp Val

3798
    GTG GAA ATT GCA CGC GCA TTG CAT CAG TTG
    Val Glu Ile Ala Arg Ala Leu His Gln Leu
```

# FIG_8

3828
```
GAC GCG GAT TCA ATT CCA GTT AAC TTC TTA
Asp Ala Asp Ser Ile Pro Val Asn Phe Leu
```

3858
```
CAT GCA ATT GAT GGA ACG AAA CTT GAA GGA
His Ala Ile Asp Gly Thr Lys Leu Glu Gly
```

3888
```
ACA CAG GAC TTA AAT CCT CGC TAT TGC TTA
Thr Gln Asp Leu Asn Pro Arg Tyr Cys Leu
```

3918
```
AAA GTA TTA GCG TTA TTC CGC TAC ATG AAT
Lys Val Leu Ala Leu Phe Arg Tyr Met Asn
```

3948
```
CCT TCG AAG GAA ATT AGA ATT TCC GGT GGT
Pro Ser Lys Glu Ile Arg Ile Ser Gly Gly
```

3978
```
CGC GAA GTC AAT TTA GGA TTC CTT CAG CCA
Arg Glu Val Asn Leu Gly Phe Leu Gln Pro
```

4008
```
TTT GGA CTG TAT GCA GCA AAT AGT ATT TTT
Phe Gly Leu Tyr Ala Ala Asn Ser Ile Phe
```

4038
```
GTT GGG GAT TAC TTA ACT ACT GAA GGA CAA
Val Gly Asp Tyr Leu Thr Thr Glu Gly Gln
```

## FIG_8

```
4068
    GAA GCC AAT AGC GAT TAT CGT ATG CTT GAA
    Glu Ala Asn Ser Asp Tyr Arg Met Leu Glu


4098
    GAT TTG GGC TTT GAA ATC GAG CTG ACA CAA
    Asp Leu Gly Phe Glu Ile Glu Leu Thr Gln


4128
    AAG CAA GAA GAA GCA TTT TGT TCT TAA TTC
    Lys Gln Glu Glu Ala Phe Cys Ser ***


4158
    AAC CAA TCA TTA TGA AAT AAA ATC TAC TAC


4188
    TAC ACA ATA TGA TTA CCT CAA AAC CGT GTG


4218                              4248
    AGCGTCGTGGAAAAGGCGCACAGACGGTTTTTTGGTCGA


             4278
    TAAAAGAGAAGGAGAAAGGTAAATAAATGGTTCCGATAATA


         4308
    TACCTATAAAATGATGGTTTTCACAAAATGTTCAATGAAA


4338                         4368
    GCGTTTTGAAATTGAACAGTTTGTGAAGGGCTTCACATAAAGCTT
```

FIG_9

FIG_10

| Plasmide | E. coli complementation | | | |
|---|---|---|---|---|
| PSBOI | D⁻ | A⁻ | B⁺ | F⁻ |
| pTG 1400 | D⁺ | A⁺ | B⁺ | F⁻ |
| pTG 1406 | D⁺ | A⁻ | B⁻ | F⁻ |
| pTG 1418 | D⁻ | A⁻ | B⁻ | F⁺ |

FIG _11

FIG _ 12

2-PvuII
2-HpaI
2-HindII
1-HpaI

1-SphI
1-KpnI
1-AvaI

1-fvvII

1-ClaI

1-HindII
1-NdeI

HindIII

1-HindIII
1    V

2-HindIII
V    4600

pBR322          NcoI          V    VV          XmnI    V          V          VV        V              V
887          1301                7187                        3361                    4525

1                              1514                    2458                    3419
1548                                  2862

EcoRI
ClaI

EcoRV
BamHI
SphI
pBR22

3419
3440
3440
3645

FIG-13

FIG_14

0266240

FIG_15

0266240

FIG_16

AAAACAATTTTAATCTACCTTCCTATCTATAAATGTGTTAACTTAATTATTATTAAGGTTAACTCAAATTGAAGAAGTTAG

1131                                        1161
AAT GGG AGG AAT AGG AGG ATG CGA AAG TTT TCT ACA TAT GAT CTT GCT
Asn Gly Arg Asn Arg Arg Met Arg Lys Phe Ser Thr Tyr Asp Leu Ala

        1191
CAG ATT TCA TTA CTA GCT TGT CTT ATT ATC GTT ACA GGC ATG
Gln Ile Ser Leu Leu Ala Cys Leu Ile Ile Val Thr Gly Met

1221                                        1251
TTT AAG ATT CCA ACA GGT ATT CCT GGA TCT GAG TTT CAA TTA TCA GCA
Phe Lys Ile Pro Thr Gly Ile Pro Gly Ser Glu Phe Gln Leu Ser Ala

        1281
CCG ATT GCC GTT GCG ATT GCA GCA GTA TTT GGA TTT AAG CGA
Pro Ile Ala Val Ala Ile Ala Ala Val Phe Gly Phe Lys Arg

1311                                        1341
TAT TTT CTT GCG GGA ATC ATT GCA AGT CTA ATC TTA TTT TTA CTA GGT
Tyr Phe Leu Ala Gly Ile Ile Ala Ser Leu Ile Leu Phe Leu Leu Gly

        1371
ATA CAC TCC ATC TTA AAT GTT GAA ATT TCA ATA ATT TTC CGA
Ile His Ser Ile Leu Asn Val Glu Ile Ser Ile Ile Phe Arg

1401                                        1431
TTG ACT GTT GGT CTA ATC ATT GTT TTA TTA GGA ACT TCA ATT CCG GTA
Leu Thr Val Gly Leu Ile Ile Val Leu Leu Gly Thr Ser Ile Pro Val

        1461
CTA GTT GTG GCA GGA CCG ATT GGA ACA ATG GTT GCT AGA CTT
Leu Val Val Ala Gly Pro Ile Gly Thr Met Val Ala Arg Leu

# Figure 17a LORF X

GGA TTG GCT TTT ACG TTA GGG ACC CCG TTT TTG CCA CTA TTC GTT TTG
Gly Leu Ala Phe Thr Leu Gly Thr Pro Phe Leu Pro Leu Phe Val Leu

GCG ATT CCA GGG ATG GTC ATT ACG GCT GTC AGT GTT TAT CCA
Ala Ile Pro Gly Met Val Ile Thr Ala Val Ser Val Tyr Pro

ATA ACG AAA ATG TTA TAT GCA ATT AAT AAG AAA GTA GCA GGT GAT CAT
Ile Thr Lys Met Leu Tyr Ala Ile Asn Lys Lys Val Ala Gly Asp His

CAT GTT AGA AAC GTG TTA TAG CAT TCG AAT GCG TGC AGC TGA
His Val Arg Asn Val Leu xxx

AAAAAATCTCGAAGGAGGAGAAAAGCATAT

# Figure 17b LORF X

TTTACGTTAGGGACCCCGTTTTTGCCACTATTCGTTTTGGCGATTCCAGGGATGGTC

ATTACGGCTGTCAGTGTTTATCCAATAACGAAAATGTTATATGCAATTAATAAGAAAGTAG

```
1618                                    1648
    CAG GTG ATC ATC ATG TTA GAA ACG TGT TAT AGC ATT CGA ATG CGT
    Gln Val Ile Ile Met Leu Glu Thr Cys Tyr Ser Ile Arg Met Arg


              1678
    GCA GCT GAA AAA AAT CTC GAA GGA GGA GAA AAG CAT ATA TCT GGT
    Ala Ala Glu Lys Asn Leu Glu Gly Gly Glu Lys His Ile Ser Gly


1708                                    1738
    GGG GAA CGG ATA GGG AGT GAA TTT CAA ATA GAG CCA ATT GTA AAA
    Gly Glu Arg Ile Gly Ser Glu Phe Gln Ile Glu Pro Ile Val Lys


              1768
    CAG TTA TTG AAC AAA GCA AGG AAT CAT TCG CGC GGA GAT GCT GAC
    Gln Leu Leu Asn Lys Ala Arg Asn His Ser Arg Gly Asp Ala Asp


1798                                    1828
    TTT ATT CAA ATT ACC GTT GAA AAA CTT ACA GGT GAT CAG ATA CTG
    Phe Ile Gln Ile Thr Val Glu Lys Leu Thr Gly Asp Gln Ile Leu


              1858
    TAT ATG CCA CCG TTA GAA ATA ACG ACA ATT GAT GAG AGT TCA ATT
    Tyr Met Pro Pro Leu Glu Ile Thr Thr Ile Asp Glu Ser Ser Ile


1888                                    1918
    GAA AGG GCA CAT AAA GAA GCT AGG AGT ATA TTA ACC TCA GTA GGT
    Glu Arg Ala His Lys Glu Ala Arg Ser Ile Leu Thr Ser Val Gly


              1948
    GTT TCC AAG CAG GCA CAA AAT GTT GCT TTT CAT CTA CTT GCT AGT
    Val Ser Lys Gln Ala Gln Asn Val Ala Phe His Leu Leu Ala Ser
```

# Figure 18a  LORF  W

1978                                          2008
AAT CAA AAT CTT CGT GGG GCT ATC CTC CTT CAT AGT CAA ACT GGC
Asn Gln Asn Leu Arg Gly Ala Ile Leu Leu His Ser Gln Thr Gly

                2038
TTA CGA CTT GAC AAT CGC GGA CTG AAA GGC GTT CGA GTA TCA CGA
Leu Arg Leu Asp Asn Arg Gly Leu Lys Gly Val Arg Val Ser Arg

2068                                    2098
ATC GAT TGG CAA GAC GCT GAT GTA GGT TAC AAT GAG CGT GTT CGT
Ile Asp Trp Gln Asp Ala Asp Val Gly Tyr Asn Glu Arg Val Arg

                2128
GAA GCG CTA GCT CTG GCA ACG AAA GTG GCA AAT TCT CCG TAT ACC
Glu Ala Leu Ala Leu Ala Thr Lys Val Ala Asn Ser Pro Tyr Thr

2158                                      2188
ATC GCA GAA TTA TGT TGG TCA GAT GAT CCA GAA TAC GTT ACT GGC
Ile Ala Glu Leu Cys Trp Ser Asp Asp Pro Glu Tyr Val Thr Gly

                2218
TAT GTA AGC AAT CAT GAG ATT GGT TAT GTC AGA ATT ACG CCT TTA
Tyr Val Ser Asn His Glu Ile Gly Tyr Val Arg Ile Thr Pro Leu

2248                                   2278
AAA AGG GAA GGC TGT GAA AGT GGC GGA CGT ATT TTT TTT GTG TCA
Lys Arg Glu Gly Cys Glu Ser Gly Gly Arg Ile Phe Phe Val Ser

                2308
GAT GAA GTT GAG CTA GAA TCA TAT ATA CAC TAT TTA GAA AGA GAA
Asp Glu Val Glu Leu Glu Ser Tyr Ile His Tyr Leu Glu Arg Glu

2338                                       2368
CCT ATT CTC ATT AGG GGG CAT TTA AAA TGA ATG ATC GCT TTC GAA
Pro Ile Leu Ile Arg Gly His Leu Lys xxx

                2398
GGG AAC TGC AAG TAA TAG AAG AGC AAG GAT TGA CAA GGA AGT TAC

2428                          2458
GTT

# Figure 18b  LORF  W

TTTTTTTGTGTCAGATGAAGTTGAGCTAGAATCATATATACACTATTTAGAAAGAGAACCTATTCTCATTAG

2352                                    2382
GGG GCA TTT AAA ATG AAT GAT CGC TTT CGA AGG GAA CTG CAA GTA
Gly Ala Phe Lys Met Asn Asp Arg Phe Arg Arg Glu Leu Gln Val

                    2412
ATA GAA GAG CAA GGA TTG ACA AGG AAG TTA CGT TTG TTT TCA ACT
Ile Glu Glu Gln Gly Leu Thr Arg Lys Leu Arg Leu Phe Ser Thr

2442                                    2472
GGA AAT GAA AGT GAG GTA GTG ATG AAT GGT AAG AAA TTT TTG CTA
Gly Asn Glu Ser Glu Val Val Met Asn Gly Lys Lys Phe Leu Leu

                    2502
TTT TCA TCG AAT AAC TAC TTA GGC CTT GCA ACA GAT AGT CGT TTG
Phe Ser Ser Asn Asn Tyr Leu Gly Leu Ala Thr Asp Ser Arg Leu

2532                                    2562
AAA AAG AAA GCA ACT GAA GGC ATT AGT AAA TAC GGT ACA GGG GCT
Lys Lys Lys Ala Thr Glu Gly Ile Ser Lys Tyr Gly Thr Gly Ala

                    2592
GGC GGT TCT CGA CTT ACA ACT GGA AAC TTC GAC ATT CAT GAA CAG
Gly Gly Ser Arg Leu Thr Thr Gly Asn Phe Asp Ile His Glu Gln

2622                                    2652
CTA GAA TCT GAA ATT GCA GAT TTT AAA AAG ACT GAA GCG GCC ATT
Leu Glu Ser Glu Ile Ala Asp Phe Lys Lys Thr Glu Ala Ala Ile

                    2682
GTA TTC AGC AGT GGG TAT TTA GCG AAC GTA GGT GTG ATT TCG AGC
Val Phe Ser Ser Gly Tyr Leu Ala Asn Val Gly Val Ile Ser Ser

2712                                    2742
GTG ATG AAG GCA GGA GAT ACT ATC TTT TCT GAT GCT TGG AAT CAC
Val Met Lys Ala Gly Asp Thr Ile Phe Ser Asp Ala Trp Asn His

Figure 19a LORF F

2772
```
GCG AGT ATT ATA GAT GGT TGT CGA TTA AGT AAA GCC AAA ACG ATT
Ala Ser Ile Ile Asp Gly Cys Arg Leu Ser Lys Ala Lys Thr Ile
```

2802                                          2832
```
GTT TAT GAA CAT GCG GAT ATG CTG GAT TTA GAG CGG AAA TTA AGG
Val Tyr Glu His Ala Asp Met Val Asp Leu Glu Arg Lys Leu Arg
```

2862
```
CAA TCA CAT GGG GAT GGA TTG AAG TTC ATC GTA ACG GAT GGC GTT
Gln Ser His Gly Asp Gly Leu Lys Phe Ile Val Thr Asp Gly Val
```

2892                                          2922
```
TTT AGT ATG GAT GGT GAT ATT GCG CCA CTT CCA AAA ATA GTA GAG
Phe Ser Met Asp Gly Asp Ile Ala Pro Leu Pro Lys Ile Val Glu
```

2952
```
TTA GCC AAG GAA TAC AAA GCG TAC ATA ATG ATT GAT GAT GCG CAT
Leu Ala Lys Glu Tyr Lys Ala Tyr Ile Met Ile Asp Asp Ala His
```

2982                                          3012
```
GCA ACA GGT GTT CTT GGC AAT GAT GGT TGT GGT ACC GCT GAT TAT
Ala Thr Gly Val Leu Gly Asn Asp Gly Cys Gly Thr Ala Asp Tyr
```

3042
```
TTT GGT TTG AAA GAT GAG ATT GAT TTT ACA GTA GGC ACG TTG AGT
Phe Gly Leu Lys Asp Glu Ile Asp Phe Thr Val Gly Thr Leu Ser
```

3072                                          3102
```
AAA GCG ATT GGT GCA GAG GGT GGA TTT GTA TCG ACA TCA TCC ATT
Lys Ala Ile Gly Ala Glu Gly Gly Phe Val Ser Thr Ser Ser Ile
```

3132
```
GCT AAG AAC TAT TTG TTA AAT AAC GCC CGA TCT TTT ATT TTC CAA
Ala Lys Asn Tyr Leu Leu Asn Asn Ala Arg Ser Phe Ile Phe Gln
```

3162                                          3192
```
ACA GCT TTA TCG CCA AGT GCG ATT GAA GCA GCG CGA GAA GGC ATT
Thr Ala Leu Ser Pro Ser Ala Ile Glu Ala Ala Arg Glu Gly Ile
```

# Figure 19b LORF F

```
                        3222
TCC ATC ATA CAG AAT GAG CCC GAG CGG AGA AAG CAA TTG CTG AAA
Ser Ile Ile Gln Asn Glu Pro Glu Arg Arg Lys Gln Leu Leu Lys


3252                                        3282
  AAT GCG CAG TAC TTA CGA TTG AAA TTA GAG GAA TCT GGT TTT GTA
  Asn Ala Gln Tyr Leu Arg Leu Lys Leu Glu Glu Ser Gly Phe Val


                        3312
ATG AAA GAA GGG GAA ACA CCT ATT ATT TCT CTT ATC ATT GGT GGT
Met Lys Glu Gly Glu Thr Pro Ile Ile Ser Leu Ile Ile Gly Gly


3342                                        3372
  TCT CAT GAA GCC ATG CAG TTT TCT GCG AAA CTA CTG GAT GAA GGT
  Ser His Glu Ala Met Gln Phe Ser Ala Lys Leu Leu Asp Glu Gly


                        3402
GTC TTT ATT CCA GCG ATT CGA CCA CCA ACA GTG CCG AAA GGG TCA
Val Phe Ile Pro Ala Ile Arg Pro Pro Thr Val Pro Lys Gly Ser


3432                                        3462
  AGT CGG TTG CGT ATA ACG GTA ATG GCT ACA CAT ACA ATA GAG CAG
  Ser Arg Leu Arg Ile Thr Val Met Ala Thr His Thr Ile Glu Gln


                        3492
CTC GAT ATG GTC ATT AGT AAA ATT AAG AAA ATA GGA AAA GAA ATG
Leu Asp Met Val Ile Ser Lys Ile Lys Lys Ile Gly Lys Glu Met


3522                                        3552
  GGG ATT GTA TAA TTG TTT GAG TGC CTG GCA CTC AAA CAA TTT TTT
  Gly Ile Val xxx


                        3582
GAT TGC TTT TTC TTA TTA ATT ACA TAG CAC TAA C
```

## Figure 19c  LORF  F

FIG. 20

pTG 1440
13112 pdb

Eco RI
CLa I
Hind III
XmnI

Pst I
— Nru I
Nde I 1606
— pTG 1400 insert —
SphI 2582
BglII 2724
Nde I 2819
HpaI - Hind II 3923
HpaI - Hind II 3945
Hind III 4407
Eco RV 4566
PBR322
10815 Pvu II
9400 Sal I - Hind II
7875 Nde I
— pTG 1418 insert —
Cal I 7052
SphI 6142
KpnI 6108
ScaI 5861
AvaI 5895

FIG _ 21

FIG_ 22

# FIG_ 23

| Plasmide | Complémentation |
|----------|-----------------|
| pTG 1418 | F⁺C⁺ |
| pTG 1433 | F⁺C⁺H⁺ |
| pTG 1434 | F⁻C⁻H⁻ |
| pTG 1435 | F⁻C⁻H⁻ |
| pTG 1436 | F⁺C⁻H⁻ |
| pTG 1437 | F⁺C⁻ |
| pTG 1439 | F⁻C⁻H⁻ |

0266240

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 93, no. 7, 18 août 1980, page 480, résumé no. 65744k, Columbus, Ohio, US; A. MUKHERJEE et al.: "Construction and characterization of a recombinant plasmid containing the entire biotin genes of E. coli K12", & PLASMIDS TRANSPOSONS, (PROC. ANNU. SYMP. SCI. BASIS MED.) 4th 1979 (Pub. 1980), 379-86 <br> * Résumé * <br> --- | 1-7,12, 20-25, 27-29 | C 12 N 15/00 <br> C 12 P 17/18 <br> C 12 N 1/00 |
| X | MOLEC. GEN. GENET., vol. 166, 1978, pages 305-312, Springer Verlag; G. COHEN et al.: "Isolation and characterization of a CoIE1 plasmid containing the entire bio gene cluster of Escherichia coli K12" <br> * En entier * <br> --- | 1-7,12, 20-25, 27-29 | |
| X | CHEMICAL ABSTRACTS, vol. 106, no. 21, mai 1987, page 183, résumé no. 170235e, Columbus, Ohio, US; & JP-A-61 202 686 (SHISEIDO CO., LTD) 08-09-1986 <br> * Résumé * <br> --- | 1-7,12, 20-25, 27-29 | |
| P,X | WO-A-8 701 391 (AMGEN) <br> * En entier * <br><br> --- | 1-7,12, 20-25, 27-29 | |
| A | J. MOL. BIOL., vol. 148, 1981, pages 63-76, Academic Press Inc. LTD, Londres, GB; A. SANCAR et al.: "Identification of the uvrB gene product" <br> * En entier * <br> ---      -/- | 1-7,12, 20,21, 24,25, 27 | |

**DOMAINES TECHNIQUES RECHERCHES (Int. Cl.4)**

C 12 N
C 12 P

Le présent rapport a été établi pour toutes les revendications

| Lieu de la recherche | Date d'achèvement de la recherche | Examinateur |
|---|---|---|
| LA HAYE | 06-01-1988 | DESCAMPS J.A. |

**Office européen des brevets**

# RAPPORT DE RECHERCHE EUROPEENNE

Numero de la demande

EP 87 40 2157

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| | GENE, vol. 1, 1977, pages 331-345, Elsevier/North-Holland Biomedical Press, Amsterdam, NL; C.K. DAS GUPTA et al.: "Isolation and characterization of the biotin genes of Escherichia coli K-12" \* En entier \* --- | 1-7,12, 20,21, 24,25, 27 | |
| A | CHEMICAL ABSTRACTS, vol. 106, no. 3, 19 janvier 1987, page 209, résumé no. 14236a, Columbus, Ohio, US; & JP-A-61 149 091 (NIPPON SODA CO., LTD) 07-07-1986 --- | | |
| A | AGRIC. BIOL. CHEM., vol. 45, no. 9, 1981, pages 1983-1989; Y. IZUMI et al.: "Characterization of biotin biosynthetic enzymes of Bacillus sphaericus: a dethiobiotin producing baterium" ----- | | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 06-01-1988 | DESCAMPS J.A. |

CATEGORIE DES DOCUMENTS CITES

X : particulièrement pertinent à lui seul
Y : particulièrement pertinent en combinaison avec un autre document de la même catégorie
. A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................
& : membre de la même famille, document correspondant

EPO FORM 1503 03.82 (P0402)